# EUROPEAN PATENT APPLICATION

(11) **EP 3 915 563 A1**
(43) Date of publication of application: **01.12.2021**
(21) Application number: 20382444.6
(22) Date of filing: 27.05.2020
(51) Int. Cl.: A61K 31/473, A61P 21/00, A61P 25/28

(54) **FOXO INHIBITORS FOR USE IN THE TREATMENT OF DISEASES CAUSED BY ABNORMAL PROCESSING OF TDP-43 AND/OR FUS PROTEINS**

(71) Applicant: Administración General de la Comunidad Autónoma de Euskadi, 01010 Vitoria-Gasteiz (Arava/Álava (ES); Universidad del País Vasco/Euskal Herriko Unibertsitatea, 48940 Leoia, Bikaia (ES)
(72) Inventor: Zufiría García, Mónica, 20014 Donostia - San Sebastián - Gipuzkoa (ES); Gereñu Lopetegi, Gorka, 20014 Donostia - San Sebastián - Gipuzkoa (ES); Gil Bea, Patxi, 20014 Donostia - San Sebastián - Gipuzkoa (ES); Alonso Martin, Sonia, 20014 Donostia - San Sebastián - Gipuzkoa (ES); Lasa Elgarresta, Jaione, 20014 Donostia - San Sebastián - Gipuzkoa (ES); López de Munain Arregui, Adolfo, 20014 Donostia - San Sebastián - Gipuzkoa (ES); Aizpurua Iparraguirre, Jesús María, Leioa, Bizkaia (ES); Miranda Murua, José Ignacio, Leioa, Bizkaia (ES)
(74) Representative: Hoffmann Eitle

(57) **Abstract**

FoxO inhibitors for use in the treatment of diseases caused by abnormal TDP-43 and/or FUS proteins. The present invention refers to FoxO inhibitors, preferably quinolone derivatives, for use in the treatment of diseases caused by alterations of TDP-43 and/or FUS proteins, preferably Amyotrophic lateral sclerosis (ALS).

SEQUENCE LISTING

<110>  Administración General de la Comunidad Autónoma de Euskadi
       UNIVERSIDAD DEL PAÍS VASCO/EUSKAL HERRIKO UNIBERTSITATEA

<120>  FOXO INHIBITORS FOR USE IN THE TREATMENT OF DISEASES CAUSED BY
       ABNORMAL PROCESSING OF TDP-43 AND/OR FUS PROTEINS

<130>  905005

<160>  6

<170>  PatentIn version 3.5

<210>  1
<211>  63
<212>  PRT
<213>  Artificial sequence

<220>
<223>  Artificial sequence

<400>  1

Lys Gly Asp Ser Asn Ser Ser Ala Gly Trp Lys Asn Ser Ile Arg His
1               5                   10                  15

Asn Leu Ser Leu His Ser Lys Phe Ile Arg Val Gln Asn Glu Gly Thr
            20                  25                  30

Gly Lys Ser Ser Trp Trp Met Leu Asn Pro Glu Gly Gly Lys Ser Gly
        35                  40                  45

Lys Ser Pro Arg Arg Arg Ala Ala Ser Met Asp Asn Asn Ser Lys
    50                  55                  60

<210>  2
<211>  61
<212>  PRT
<213>  Artificial sequence

<220>
<223>  Artificial sequence

<400>  2

Lys Gly Asp Ser Asn Ser Ser Ala Gly Trp Lys Asn Ser Ile Arg His
1               5                   10                  15

Asn Leu Ser Leu His Asn Arg Phe Met Arg Val Gln Asn Glu Gly Thr
            20                  25                  30

Gly Lys Ser Ser Trp Trp Met Leu Asn Pro Glu Ala Lys Pro Gly Lys
        35                  40                  45

Ser Val Arg Arg Arg Ala Ala Ser Met Glu Thr Ser Arg

50                  55                  60

<210>  3
<211>  63
<212>  PRT
<213>  Artificial sequence

<220>
<223>  Artificial sequence

<400>  3

Lys Gly Asp Ser Asn Ser Ser Ala Gly Trp Lys Asn Ser Ile Arg His
1               5                   10                  15

Asn Leu Ser Leu His Ser Lys Phe Ile Arg Val Gln Asn Glu Gly Thr
            20                  25                  30

Gly Lys Ser Ser Trp Trp Met Leu Asn Pro Glu Gly Gly Lys Ser Gly
        35                  40                  45

Lys Ser Pro Arg Arg Arg Ala Ala Ser Met Asp Asn Asn Ser Lys
    50                  55                  60

<210>  4
<211>  63
<212>  PRT
<213>  Artificial sequence

<220>
<223>  Artificial sequence

<400>  4

Lys Gly Asp Ser Asn Ser Ser Ala Gly Trp Lys Asn Ser Ile Arg His
1               5                   10                  15

Asn Leu Ser Leu His Ser Lys Phe Ile Arg Val Gln Asn Glu Gly Thr
            20                  25                  30

Gly Lys Ser Ser Trp Trp Met Leu Asn Pro Glu Gly Gly Lys Ser Gly
        35                  40                  45

Lys Ser Pro Arg Arg Arg Ala Ala Ser Met Asp Asn Asn Ser Lys
    50                  55                  60

<210>  5
<211>  63
<212>  PRT
<213>  Artificial sequence

<220>
<223>  Artificial sequence

<400>  5

Lys Gly Asp Ser Asn Ser Ser Ala Gly Trp Lys Asn Ser Ile Arg His
1               5                   10                  15

Asn Leu Ser Leu His Ser Lys Phe Ile Arg Val Gln Asn Glu Gly Thr
            20                  25                  30

Gly Lys Ser Ser Trp Trp Met Leu Asn Pro Glu Gly Gly Lys Ser Gly
        35                  40                  45

Lys Ser Pro Arg Arg Arg Ala Ala Ser Met Asp Asn Asn Ser Lys
    50                  55                  60

<210>  6
<211>  63
<212>  PRT
<213>  Artificial sequence

<220>
<223>  Artificial sequence

<400>  6

Lys Gly Asp Ser Asn Ser Ser Ala Gly Trp Lys Asn Ser Ile Arg His
1               5                   10                  15

Asn Leu Ser Leu His Ser Lys Phe Ile Arg Val Gln Asn Glu Gly Thr
            20                  25                  30

Gly Lys Ser Ser Trp Trp Met Leu Asn Pro Glu Gly Gly Lys Ser Gly
        35                  40                  45

Lys Ser Pro Arg Arg Arg Ala Ala Ser Met Asp Asn Asn Ser Lys
    50                  55                  60

## Description

### FIELD OF THE INVENTION

The present invention refers to the medical field. Particularly, the present invention refers to FOXO inhibitors, preferably quinolone derivatives, for use in the treatment of diseases caused by alterations of TDP-43 and/or FUS proteins, preferably Amyotrophic lateral sclerosis (ALS) and Frontotemporal dementias.

### PRIOR ART

TDP-43 and FUS are both RNA/DNA-binding proteins with striking structural and functional similarities. They have been associated with ALS and other neuromuscular and/or neurodegenerative disorders such as: Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy (see references 1 to 13).

Particularly, ALS is a devastating disorder characterized by a progressive degeneration of upper and lower motor neurons that inevitably leads to the death of the patient within an average of 36 months from onset. Major symptoms, including weakness, motor disability, paralysis and ultimately death arise from the progressive wasting of skeletal muscle. Although motor neuron loss and subsequent dismantlement of neuromuscular junctions are considered the primary cause of muscle atrophy, recent studies have challenged this view by showing clear evidence of early muscle metabolic alterations in relation to disease progression, occurring even prior to muscle denervation in SOD1 mouse models.

Maintenance of muscle fibre homeostasis in adulthood is fundamentally supported by intrinsic repairing mechanisms that involve a sequence of metabolically fine-tuned steps; starting from the activation and proliferation of skeletal muscle resident stem cells (known as satellite cells) until the differentiation and fusion of myogenic myoblasts into new myofibers. Several lines of evidence have brought into light that muscle regeneration is affected in ALS, and that impaired myogenic processes would aggravate the denervation-induced muscle wasting. In this sense, satellite cells isolated from patients with ALS have shown changes in various myogenic markers and impaired ability to form mature myotubes in *in vitro* conditions. Likewise, myogenic defects have been also reported in cultured myoblasts derived from mouse models expressing mutations in either SOD1 or VAPB. Although these myogenic defects may be legitimately considered secondary to changes in the denervated muscle, specially the stem cell niche, or an intrinsic defect in muscle stem cells, recent discovery of the moonlighting activity of *TARDBP* (a major ALS-causative gene) in muscle regeneration have bolstered the argument for the implication of myogenesis in the pathophysiology of ALS. The product of *TARDBP* gene known as TDP-43 was proved essential for normal muscle formation and regeneration.

Since there is an unmet medical need of finding reliable treatments for diseases caused by alterations of TDP-43 and/or FUS proteins, such as ALS, the inventors of this patent application are seeking for pathological mechanisms underlying these abnormalities, and a new approach for treating said diseases is herein provided.

### DESCRIPTION OF THE INVENTION

### Brief description of the invention

The inventors of the present invention have studied the contribution of two major ALS genes, including *TARDBP* and *FUS*, in the metabolic regulation of myogenesis and muscle regeneration, by using human immortalized myoblasts with induced loss-of-function of these genes, primary myoblasts from patients with ALS, as well as *Drosophila* models. Metabolic flux analyses enabled the discovery of repressed glycolysis as a consistent metabolic feature linking energy defects with the impaired myogenic capacity induced by TDP-43 and FUS knockdown.

In addition, biochemical and gene expression surveys led to the identification of FoxO (Forkhead Box O) transcription factors as potential regulators of the differentially expressed genes in TDP-43 and FUS knockdown myoblasts.

Interestingly, pharmacological and genetic inhibition of FoxO alleviated the metabolic defects and corrected the myogenic differentiation ability in the immortalized myoblasts with induced deficiency of TDP-43 and FUS. The metabolic and myogenic abnormalities of silenced myoblasts were reproduced in primary myoblasts derived from two patients with ALS, and consequently alleviated by FoxO inhibition. Finally, in *Drosophila* models that bear a conditional knockdown of the fly gene orthologs for *TARDBP* and *FUS*, *Tbph* and *Caz*, respectively, in muscle progenitor cells, impaired motor function and decreased lifespan were found, both of them alleviated by pharmacological inhibition of FoxO.

So, in summary, the inventors of this patent application have identified that FoxO factors are mediators of the effects of TDP-43 or FUS loss-of-function on metabolic and functional stasis in skeletal muscle.

Therefore, FoxO, preferably FoxO1 (Entrez Gene: 2308; Ensembl: ENSG00000150907; OMIM: 136533) and FoxO3 (Entrez Gene: 2309 Ensembl: ENSG00000118689 OMIM: 602681) is herein proposed as a therapeutic target for the treatment of diseases caused by alterations of TDP-43 and/or FUS proteins, preferably for the treatment of ALS.

So, the first embodiment of the present invention refers to FoxO inhibitors for use in the treatment of neuromuscular and/or neurodegenerative disorders. Alternatively, this first embodiment refers to a method for treating neuromuscular and/or neurodegenerative disorders which comprises the administration of a therapeutically effective dose or amount of a FoxO inhibitor, or a pharmaceutical composition comprising FoxO inhibitors and, optionally, pharmaceutically acceptable excipients or carriers.

### Diseases to be treated according to the present invention

In a preferred embodiment, the neuromuscular and/or neurodegenerative disorders to be treated according to the present invention are selected from the group of diseases caused by alterations of TDP-43 and/or FUS proteins, preferably from the group comprising: ALS, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

In a particularly preferred embodiment the disease is ALS.

### FoxO inhibitors

Such as it is demonstrated in the results included in this patent application, the contribution that the inventors have made over the prior art is the identification of FoxO as a therapeutic target for the treatment of the above cited diseases, particularly ALS, irrespective of the FoxO inhibitor which is finally used, no matter if the inhibitor is a quinolone derivative, a biological molecule such a siRNA, a miRNA (such as miR-205-5p) or an antibody, or other compounds like carbenoxolone and benserazide.

According to the present invention, the compound 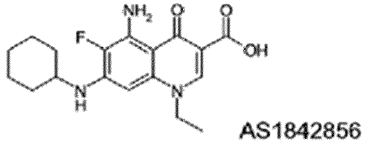 and a shRNA sequence (TRCN0000010333, human FOXO MISSION shRNA, MISSION® pLKO.1-puro, Sigma Aldrich), which targets mRNA of both FoxO1 and FoxO3, are used as proof-of-concept FoxO inhibitors, demonstrating that both the pharmaceutical and genetic inhibition of FoxO is effective.

In a preferred embodiment, FoxO inhibitor is a quinolone derivative, which comprises within its structure a quinolone, for example:

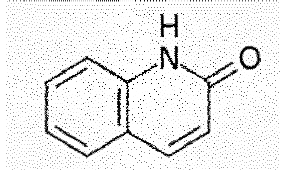

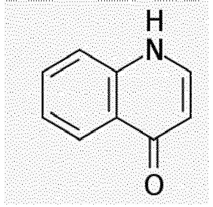

By way of example, any quinolone derivative included in the patent application US20090197834A1 is herein included by reference, particularly a quinolone derivative represented by the following formula or a pharmaceutically acceptable salt thereof: 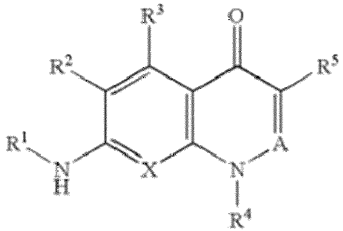 wherein
R¹: a cycloalkyl or lower alkylene-cycloalkyl, wherein the cycloalkyl in R¹ may be substituted;
R²: -H or a halogen;
R³: -H, a halogen, -OR⁰ or -O-lower alkylene-aryl;
R⁰: the same or different from each other and each represents -H or a lower alkyl
R⁴: a lower alkyl, halogeno-lower alkyl, lower alkylene-cycloalkyl, cycloalkyl or heterocyclic group, wherein the cycloalkyl and heterocyclic group in R⁴ may respectively be substituted;
R⁵: -NO₂, -CN, a lower alkyl, lower alkenyl, halogeno-lower alkenyl, -L-R^{a}, -C(O)R⁰, -O-R^{b}, -N(R⁶)₂, lower alkylene-N(R⁶)(R^{c}), -N(R⁶)C(O)-R^{d}, lower alkylene-N(R⁶)C(O)-R^{d}, lower) alkylene-N(R⁰)C(O)O-lower alkyl, -N(R⁰)C(O)N(R⁰)-R^{e}, lower alkylene-N(R⁰)C(O)N(R⁰)-R^{e}, -N(R⁰)S(O)₂N(R⁰)C(O)-R^{d}, -CH=NOH, cycloalkyl, heterocyclic group, (2,4-dioxo-1,3-thiazolidin-5-ylidene)methyl or (4-oxo-2-thioxo-1,3-thiazolidin-5-ylidene)methyl, wherein the cycloalkyl and heterocyclic group in R⁵ may respectively be substituted;
R⁶: H, a lower alkyl, lower alkylene-CO₂R⁰ or lower alkylene-P(O)(OR^{p})₂, wherein the lower alkylene in R⁶ may be substituted;
L: a lower alkylene or lower alkenylene which may respectively be substituted;
R^{a}: -OR⁰, -CN, -O-lower alkylene-aryl, -O-lower alkylene-CO₂R⁰, -C(O)R⁰,-CO₂R⁰, -C(O)NHOH, -C(O)N(R⁶)₂, -C(O)N(R⁰)-aryl, -C(O)N(R⁰)-S(O)₂-lower alkyl, -C(O)N(R⁰)-S(O)₂-aryl, -C(O)N(R⁰)-S(O)₂-heterocyclic group, -NH₂OH,-OC(O)R⁰, -OC(O)-halogeno-lower alkyl, -P(O)(OR^{p})₂, an aryl or heterocyclic group, wherein the aryl and heterocyclic group in R^{a} may be substituted;
R^{p}: R⁰, a lower alkylene-OC(O)-lower alkyl, lower alkylene-OC(O)-cycloalkyl, lower alkylene-OC(O)O-lower alkyl, lower alkylene-OC(O)O-cycloalkyl, or lower alkylene-heterocyclic group, wherein the heterocyclic group in R^{p} may be substituted;
R^{b}: H, a cycloalkyl, aryl, heterocyclic group, lower alkylene-R^{ba} or lower alkenylene-R^{ba}, wherein the lower alkylene, lower alkenylene, cycloalkyl, aryl and heterocyclic group in R^{b} may be substituted;
R^{ba}: -OR⁰, -O-Si(lower alkyl)₃, -CO₂R⁰, -C(O)NHOH, -C(O)N(R⁰)₂,-C(O)N(R⁰)-S(O)₂-lower alkyl, -C(O)N(R⁰)-S(O)₂-aryl, =C(NH₂)=NOH,-C(NH₂)=NO-C(O)R⁰, -C(NH₂)=NO-C(O)-lower alkylene-C(O)R⁰, -CO₂-lower alkylene-aryl, -P(O)(OR^{p})₂, -C(O)R⁰, -C(O)-aryl, a cycloalkyl, aryl or heterocyclic group, wherein the aryl and heterocyclic group in R^{ba} may be substituted;
R^{c}: H, a lower alkyl, lower alkylene-OR⁰, lower alkylene-CO₂R⁰, lower alkylene-C(O)NHOH, lower)alkylene-C(O)N(R⁰)₂, lower alkylene-P(O)(OR^{P})₂, lower alkylene-aryl, lower alkylene-heterocyclic group, aryl or heterocyclic group, wherein the lower alkylene, aryl and heterocyclic group in R^{c} may be substituted;
R^{d}: a C₁₋₇ alkyl, lower alkenyl, halogeno-lower alkyl, lower alkylene-R^{da}, lower alkenylene-R^{da}, cycloalkyl, aryl or heterocyclic group, wherein the lower alkylene, lower alkenylene, cycloalkyl, aryl and heterocyclic group in R^{d} may be substituted;
R^{da}: -CN, -OR⁰, -OC(O)R⁰, -O-lower alkylene-CO₂R⁰, -O-aryl, -CO₂R⁰,-C(O)NHOH, -C(O)N(R⁰)₂, -CO₂-lower alkylene-N(R⁰)₂, -P(O)(OR^{p})₂, -N(R⁶)₂,-N(R⁰)C(O)R⁰, -C(O)N(R⁰)-aryl, -C(O)N(R⁰)-(lower alkylene which may be substituted with -CO₂R⁰)-aryl, -N(R⁰)C(O)-aryl, -N(R⁰)C(O)-OR⁰, -N(R⁰)C(O)-O-lower alkylene-aryl, -N(R⁰)S(O)₂-aryl, -S-heterocyclic group, -C(O)N(R⁰)-heterocyclic group, -N(R⁰)C(O)-heterocyclic group, a cycloalkyl, aryl or heterocyclic group, wherein the cycloalkyl, aryl and heterocyclic group in R^{da} may be substituted;
R^{e}: lower alkylene-CO₂R⁰, lower alkylene-C(O)NHOH, lower)alkylene-C(O)N(R⁰)₂, a lower alkylene-heterocyclic group, aryl, heterocyclic group, -S(O)₂-aryl or -S(O)₂-heterocyclic group, wherein the aryl and heterocyclic group in R^{e} may be substituted;
X: CH;
A: C(R⁷);
R⁷: -H or a lower alkyl, or R⁴ and R⁷ may together form a lower alkylene which may be substituted.

In a particularly preferred embodiment, the quinolone derivative is selected from the group comprising:

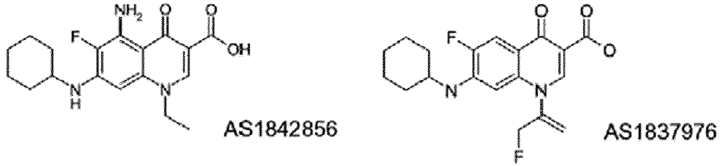

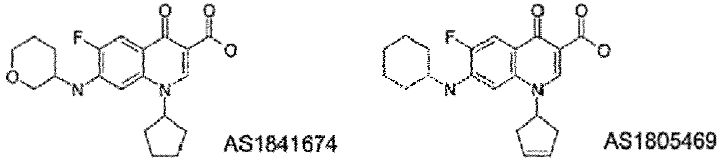

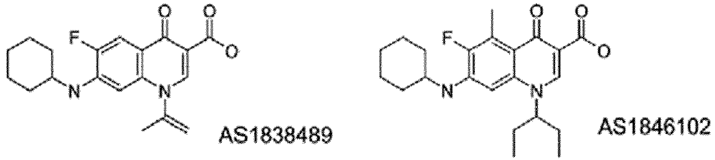

In a particularly preferred embodiment the FoxO inhibitor is

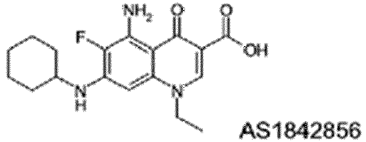

More examples of FoxO inhibitors which could be used according to the present invention are:

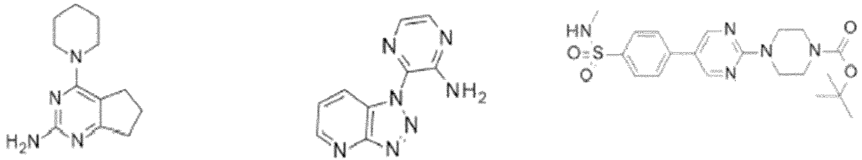

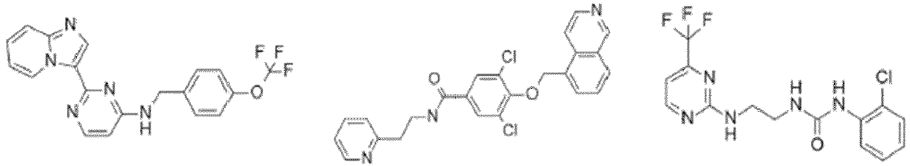

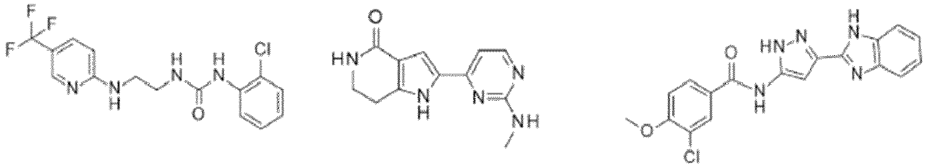

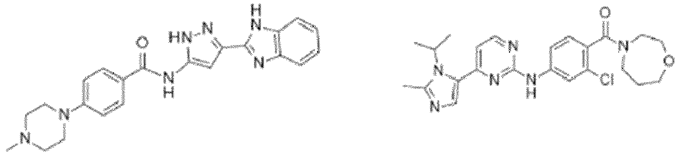

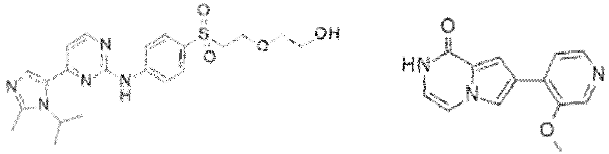

### Pharmaceutical composition

The second embodiment of the present invention refers to a pharmaceutical composition comprising any of the FoxO inhibitors described above, or pharmaceutically acceptable salt thereof and, optionally, pharmaceutically acceptable excipients or carriers, for use in the treatment of any of the above mentioned neuromuscular and neurodegenerative disorders, particularly ALS.

### Screening method

The third embodiment of the present invention refers to an *in vitro* method for the identification and production of candidate compounds for the treatment of Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, comprising: a) determining whether FoxO has been inhibited after administering the candidate molecule, b) where, if after administering the candidate molecule, FoxO has been inhibited, this is indicative that the candidate molecule is effective in the treatment of Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

In a preferred embodiment, the method is directed to the identification and production of candidate compounds for the treatment of ALS, comprising: a) determining whether FoxO has been inhibited after administering the candidate molecule, b) where, if after administering the candidate molecule, FoxO has been inhibited, this is indicative that the candidate molecule is effective in the treatment of Amyotrophic Lateral Sclerosis.

### Method for monitoring the response to the treatment using FoxO as biomarker

The fourth embodiment of the present invention refers to an *in vitro* method for monitoring the response to a treatment against ALS, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, comprising: a) determining whether FoxO has been inhibited after administering the treatment, b) where, if after administering the treatment, FoxO has been inhibited, this is indicative that the treatment is effective in the treatment of Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

In a preferred embodiment, the method is for monitoring the response to a treatment against ALS, comprising: a) determining whether FoxO has been inhibited after administering the treatment, b) where, if after administering the treatment, FoxO has been inhibited, this is indicative that the treatment is effective in the treatment of ALS.

### Diagnostic method using FoxO as biomarker

The fifth embodiment of the present invention refers to an *in vitro* method for the diagnosis of neuromuscular and neurodegenerative disorders selected from the group comprising: ALS, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, which comprises measuring the level of expression of FoxO in a biological sample obtained from a patient, wherein a variation or deviation of the level of expression of FoxO measured in the patient with respect to the level measured in healthy control patients, it is indicative that the patient is suffering from a neuromuscular and neurodegenerative disorder selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

### Diagnostic method using NAD⁺ (or NAD⁺/NADH index) as biomarker

On the other hand, according to the present invention, the decrease of NAD+ (or NAD⁺/NADH index) in biological samples could be an indication of any of the above defined diseases, particularly ALS. In other words, NAD⁺ (or NAD⁺/NADH index) could be used as a biomarker for the diagnosis of any of the above defined diseases, particularly ALS.

The inventors have shown that the inhibition of FoxO would induce a recovery of glycolitic function and a secondary recovery of cytosolic NAD⁺.

In other words, since the inhibition of FoxO is herein presented as a treatment of the above defined diseases, and the inhibition of FoxO would induce a recovery of cytosolic NAD⁺, the decrease of NAD⁺ (or NAD⁺/NADH index) would be an indication that the patient is suffering from any of the above cited diseases, particularly ALS.

So, the sixth embodiment of the present invention refers to an *in vitro* method for the diagnosis of neuromuscular and neurodegenerative disorders selected from the group comprising: ALS, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, which comprises measuring the level of expression of NAD⁺ (or NAD⁺/NADH index) in a biological sample obtained from the patient, wherein a reduction of the level of expression of NAD⁺ (or NAD⁺/NADH index) measured in the patient with respect to the level measured in healthy control patients, is indicative that the patient is suffering from a neuromuscular and neurodegenerative disorders selected from the group comprising: ALS, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

### Method for predicting or monitoring the response to a treatment using NAD⁺ (or NAD⁺/NADH index) as biomarker

Conversely, a normal or increased level of NAD⁺ (or NAD⁺/NADH index) would be an indication that the patient is not suffering from the above defined diseases, particularly ALS. So, in other words, the increase/normalized level of NAD⁺ (or NAD⁺/NADH index) would be an indication that the treatment against the above cited diseases is effective.

The seventh embodiment of the present invention refers to an *in vitro* method for predicting or monitoring the response to a treatment of a neuromuscular and neurodegenerative disorders selected from the group comprising: ALS, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, which comprises measuring the level of expression of NAD⁺ (or NAD⁺/NADH index) in a biological sample obtained from the patient after the treatment, wherein an increase of the level of expression of NAD⁺ (or NAD⁺/NADH index) measured in the patient with respect to the level measured in control patients, is indicative that the treatment is effective.

In a preferred embodiment, the above cited methods can be carried out in a biological sample selected from: urine, plasma, tears or cerebrospinal fluid (CSF).

For the purpose of the present invention the following terms are defined:
- The term "comprising" is meant including, but not limited to, whatever follows the word "comprising". Thus, use of the term "comprising" indicates that the listed elements are required or mandatory, but that other elements are optional and may or may not be present.
- By "consisting of' is meant including, and limited to, whatever follows the phrase "consisting of'. Thus, the phrase "consisting of' indicates that the listed elements are required or mandatory, and that no other elements may be present.
- By "therapeutically effective dose or amount" is intended an amount that, when administered as described herein, brings about a positive therapeutic response in the patient. The exact amount required will vary from subject to subject, depending on (non-exhaustive list): the species, age, general condition of the subject, the severity of the condition being treated or the mode of administration. An appropriate "effective" amount in any individual case may be determined by one of ordinary skill in the art using routine experimentation, based upon the information provided herein.
- The expression "reference concentration level measured in healthy control subjects", refer to a "reference value" of the concentration level of the biomarkers. If a deviation of the level of the biomarkers is determined with respect to said "reference concentration level measured in healthy control subjects", this is an indication of the disease.
- A "reference value" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative).
   Preferably, the person skilled in the art may compare the biomarker levels (or scores) obtained according to the method of the invention with a defined threshold value. Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtaining a classification standard having significance for sample classification. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then, sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches to 1.0. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software or SPSS 9.0.

### Brief description of the figures

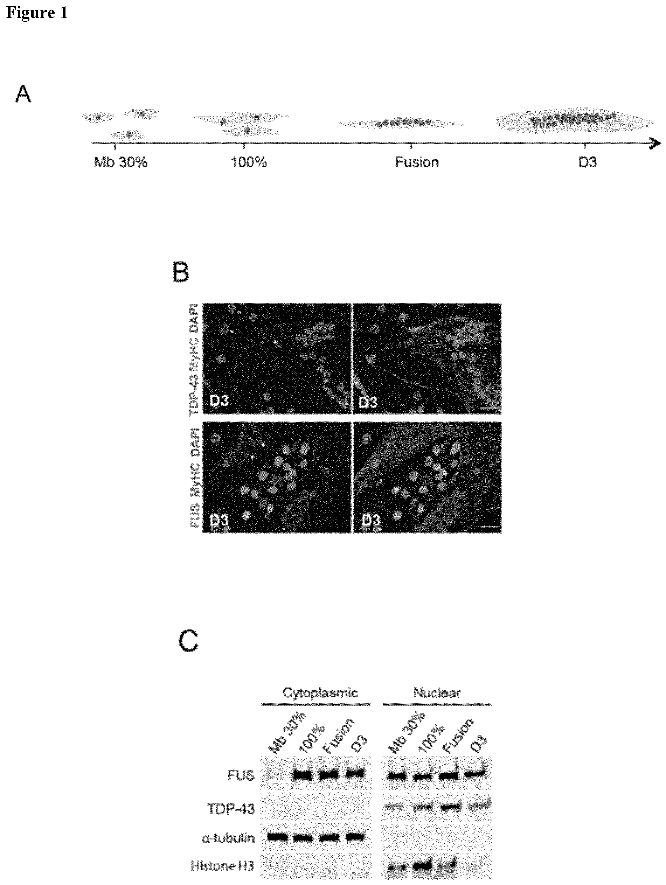

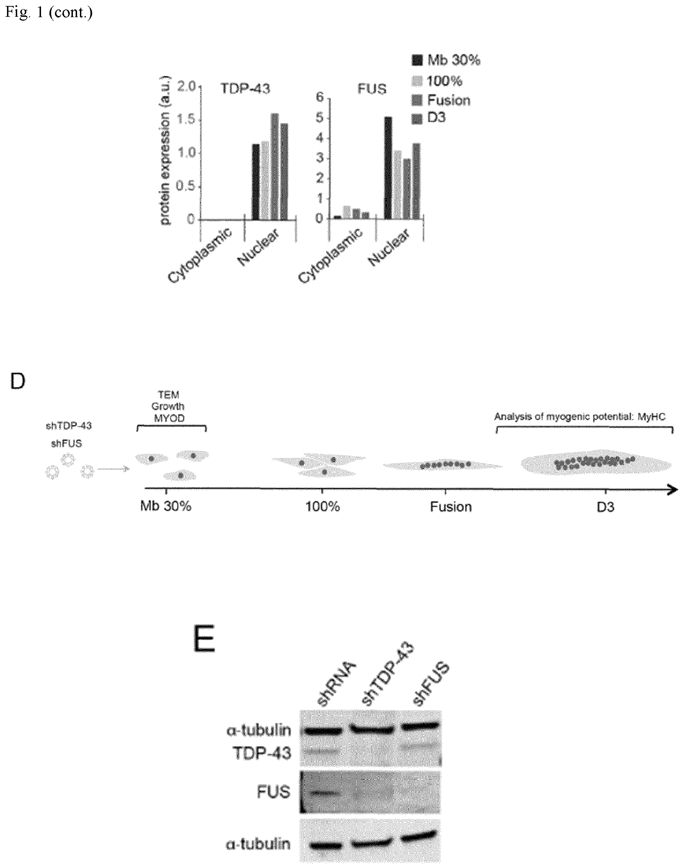

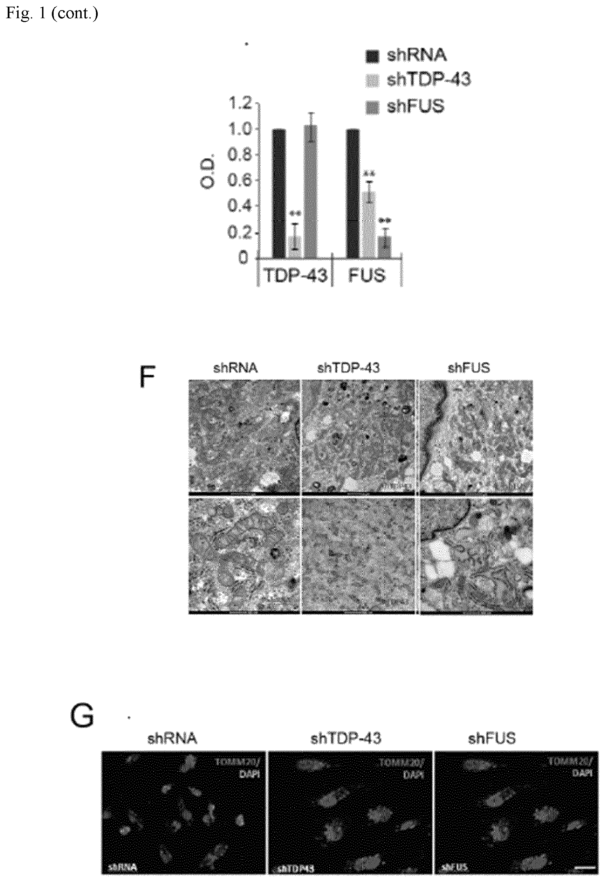

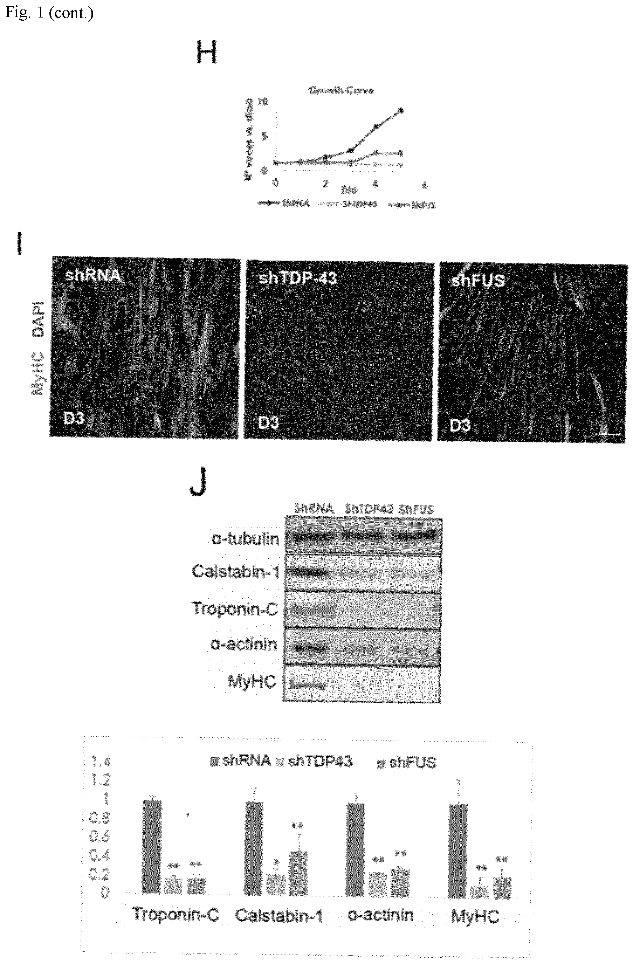

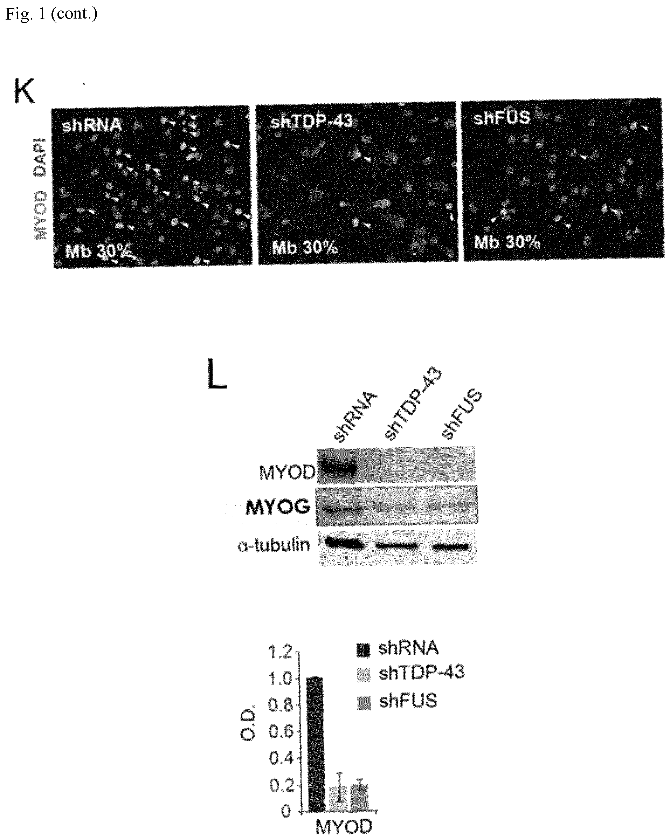

**Figure 1****. Deficiency of TDP-43 or FUS halts the myogenic capacity of 8220 human immortalized myoblasts. A**, Schematic illustration of the myogenic process (Mb, myoblasts; D3, differentiation day 3). **B-C**, Subcellular localization of TDP-43 and FUS throughout stages of myoblast differentiation (n=2). **D-E**, Lentiviral transduction of shTDP-43 and shFUS induced silencing of either proteins (n=3). **F**, Ultrastructure of silenced myoblasts observed by transmission electron microscopy. **G**, Mitochondrial network of silenced myoblasts as evidenced by immunostaining with mitochondria marker TOMM20. H, silenced myoblasts show decreased growing rates. I, shTDP-43 and shFUS-treated myoblasts have abnormal myogenic capacity, as evidenced by lower MYHC-positive myotubes. **J**, Effects of shTDP-43 and shFUS treatment on markers of myogenesis (n=2). **K-L**, Western-blot and immunofluorescence analysis reveal that shTDP-43 and shFUS-treated myoblasts have reduced levels of the myogenic markers MYOD and Myogenin (n=3). Mb, myoblasts; D3, differentiation day 3; MYHC, Myosin Heavy Chain protein (T-test. **p*<0.01; ***p*<0.01).

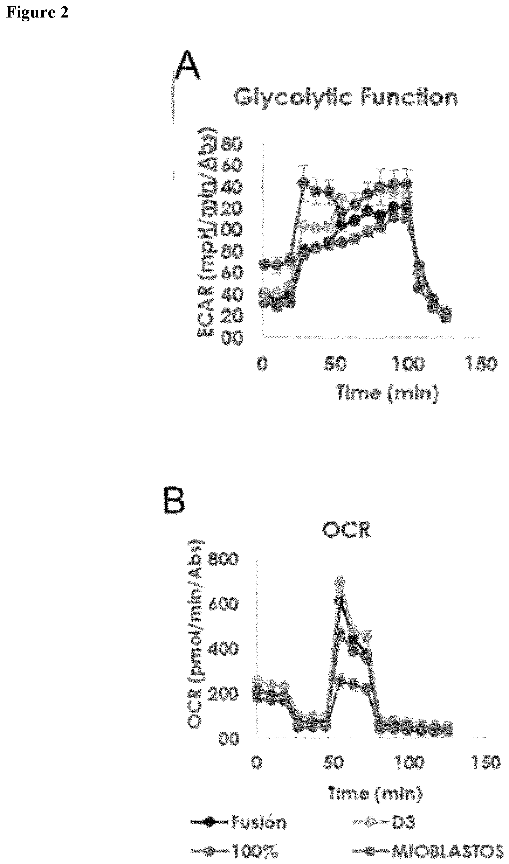

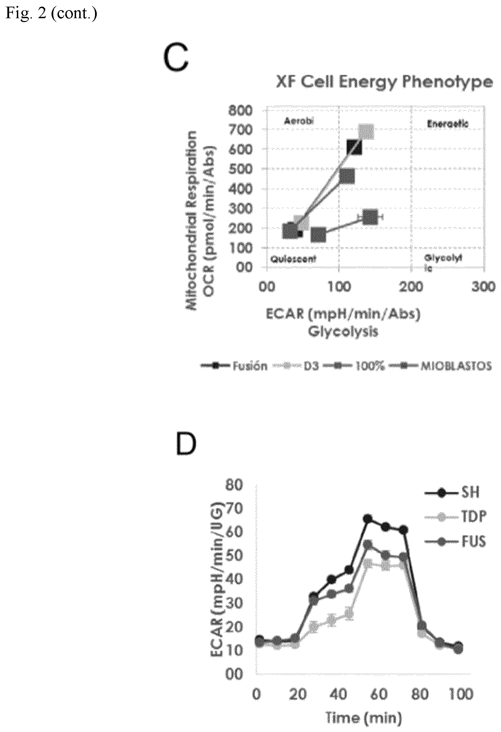

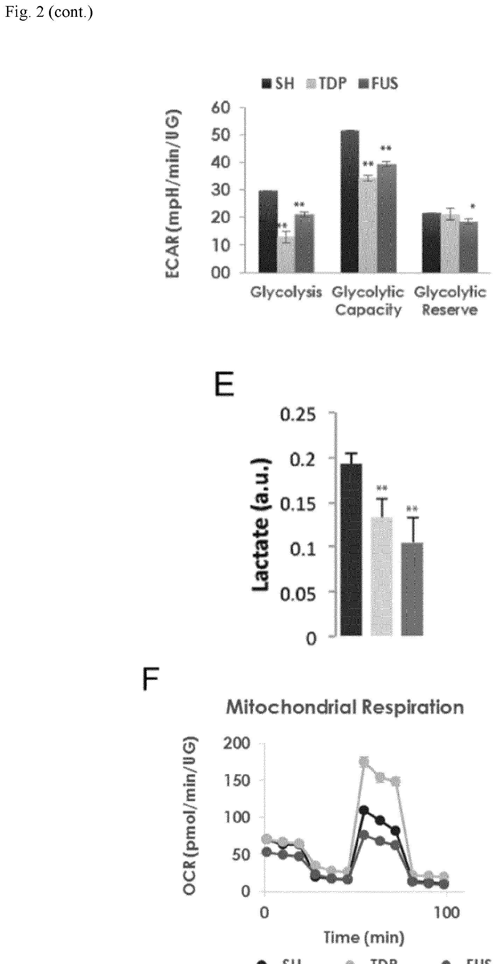

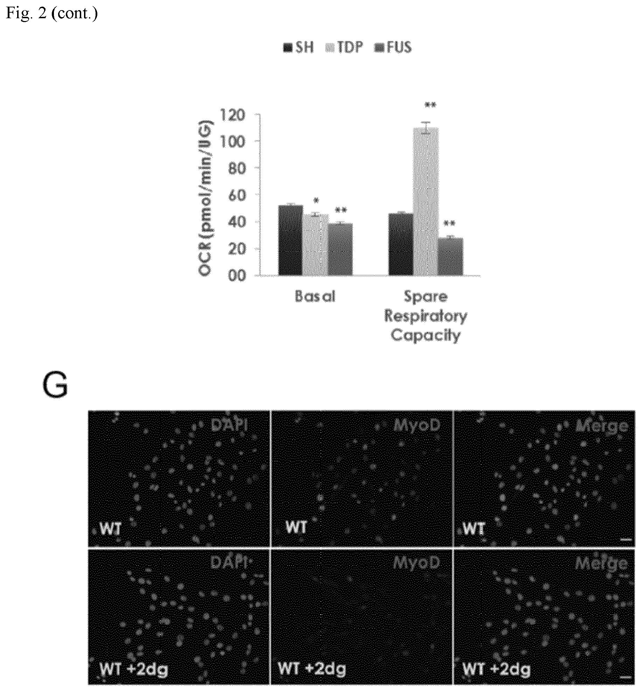

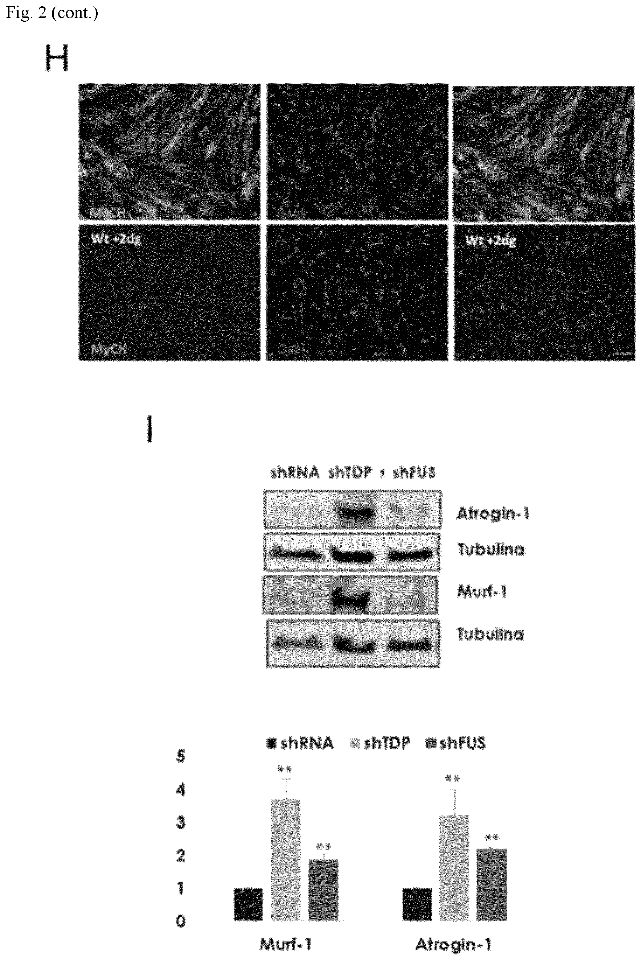

**Figure 2****. Deficiency of TDP-43 or FUS curtails anaerobic glucose metabolism and induces pro-atrophic pathways in myoblasts. A-B-C**, Metabolic switch during myogenesis. Myoblasts rely mainly on anaerobic glycolysis while differentiating myocytes and myotubes switch towards a more aerobic metabolism (n=6 experiments). **D**, shTDP-43 and shFUS-treated myoblasts display consistent reductions in basal glycolysis and glycolytic capacity (n=6 experiments). **E**, Levels of lactate are decreased in shTDP-43 and shFUS-treated myoblasts (n=3). **F**, Basal mitochondrial ATP production is slightly affected by TDP-43 or FUS silencing, however effects on spare respiratory capacity are variable (n=6 experiments). **G**, Immunofluorescence and immublot images of MyoD in 2dg-treated myoblasts. **H**, Immunostaining images showing the effect of 2dg on the number of MyHC-postive myotubes. **I**, Pro-atrophic pathways of the Unfolded Protein System (UPS) are dramatically augmented in shTDP-43 and shFUS-treated myoblasts (n=3). ECAR, Extracellular Acidification Rate; OCR, Oxygen Consumption Rate: 2dg, 2-deoxyglucose. T-test, **p*<0.05; ***p*<0.01.

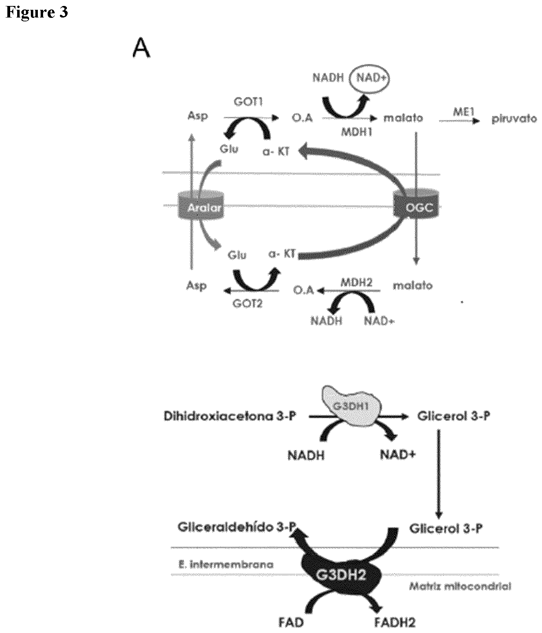

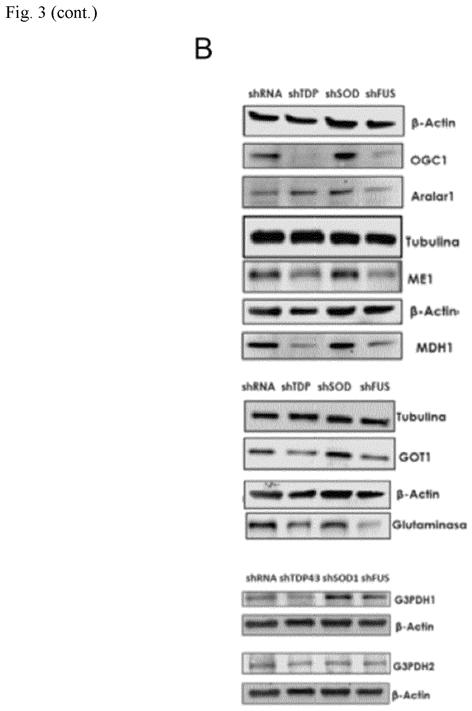

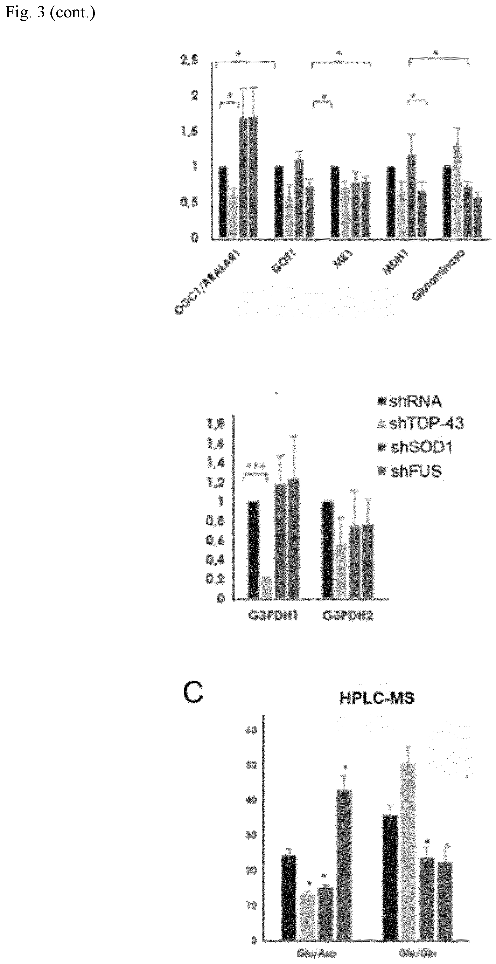

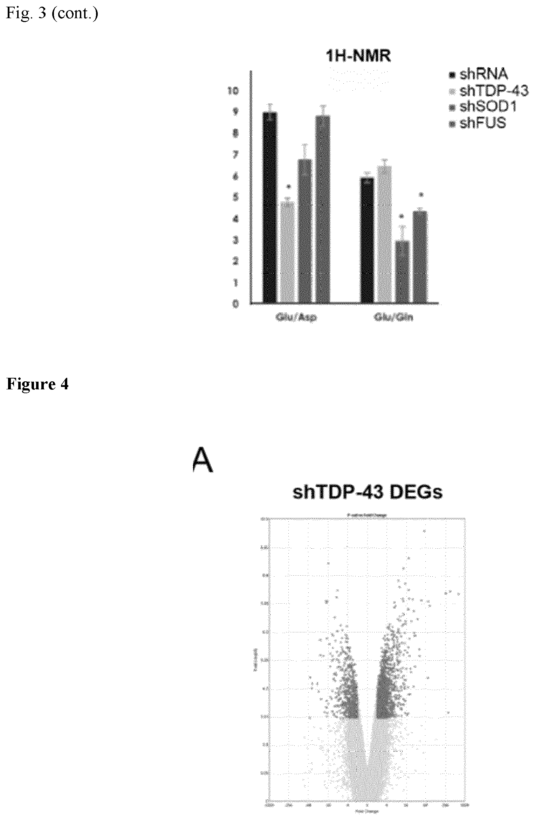

**Figure 3****. Impaired NAD⁺ recycling contributes to the glycolytic defects associated to TDP-43 and FUS deficiency. A**, Schematic illustration of malate-aspartate (upper) and glycerol-phosphate (lower) shuttles that recycle cytosolic NAD⁺. **B**, Effects of TDP-43 and FUS deficiency on protein levels of enzymes that participate in malate-aspartate and glycerol-phosphate shuttles as well as in glutaminolysis (n=6). **C**, Glutamate/Aspartate (Glu/Asp) and Glutamate/Glutamin (Glu/Gln) ratios in TDP-43 and FUS-silenced myoblasts, as measured by HPLC-MS (left panel) or ¹H-NMR (right panel) (n=6). ARALAR1, mitochondrial aspartate-glutamate carrier. T-test, **p*<0.05; ****p*<0.001.

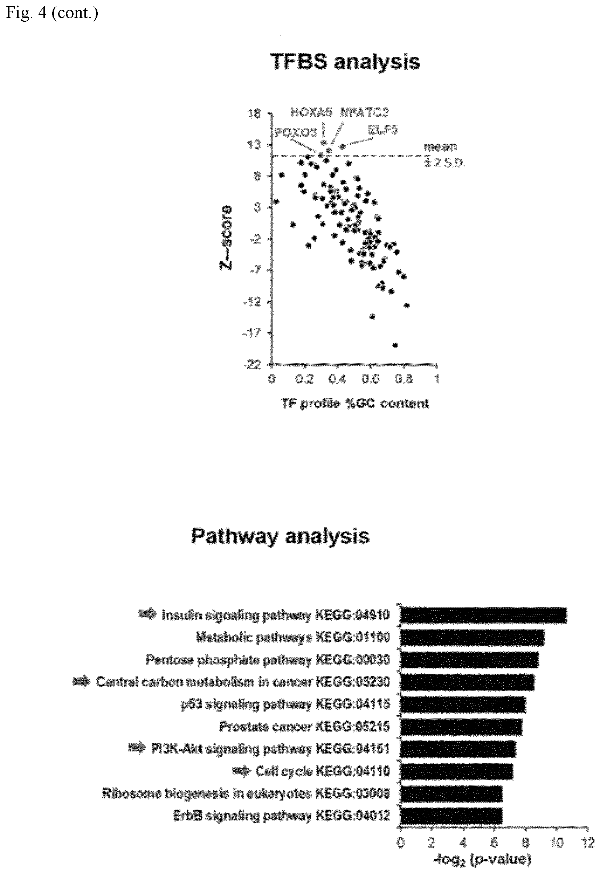

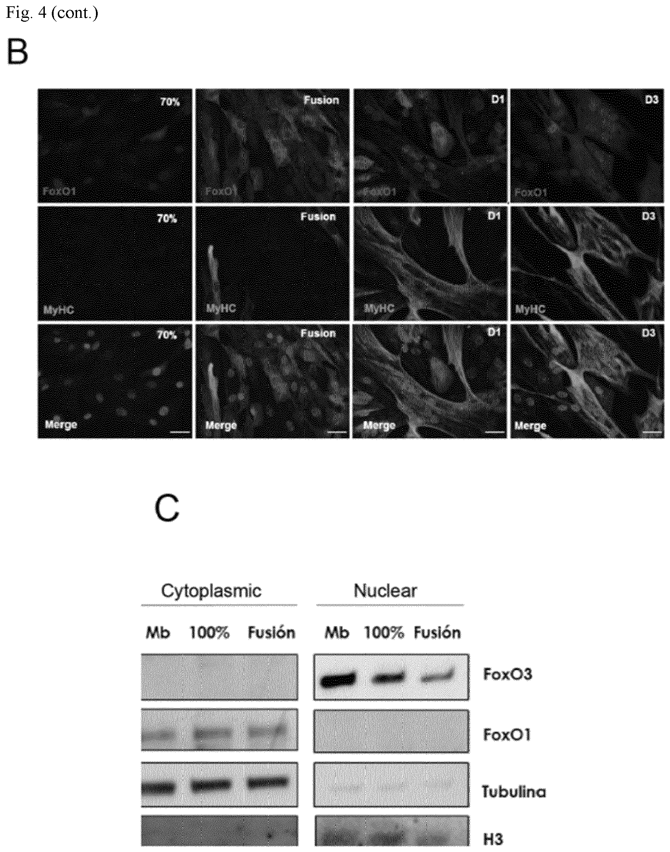

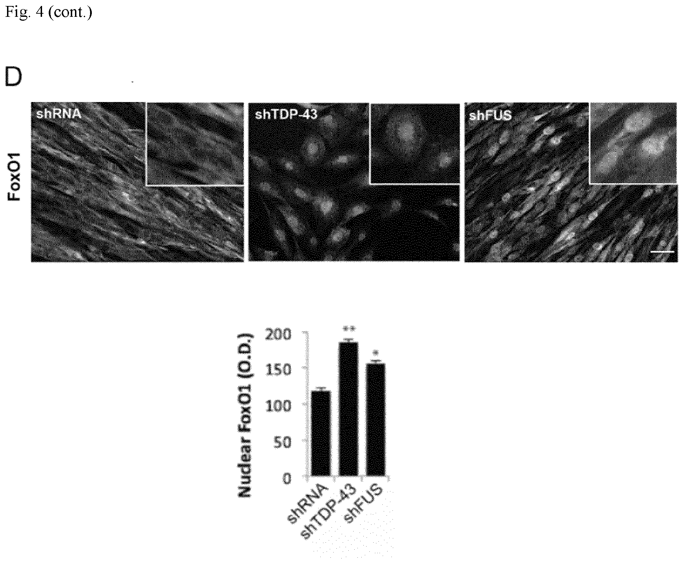

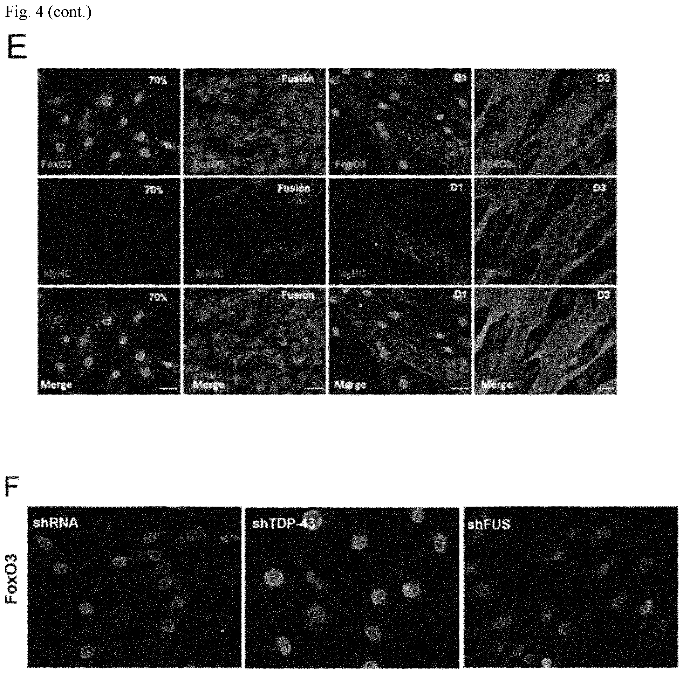

**Figure 4****. Deficiency of TDP-43 or FUS induces dysregulation of FoxO transcription factors**. **A**, Microarray of gene expression shows 1,329 upregulated genes while 673 downregulated genes in TDP-43-silenced myoblasts. Using oPOSSUM-3 online tool, FoxO3 was found among the top candidates predicting the differentially expressed genes (DEGs) in shTDP-43-treated myoblasts. Gene ontology (GO) analysis in shTDP-43 knockdown myoblasts revealed a significant enrichment of KEGG pathways that are upstream or downstream FoxO transactivation, such as insulin and PI3K-AKT signalling or cell cycle. **B and E**, Immunofluorescence images of FoxO1 (B) and Fox03 staining (E) in wild-type human myoblast during different states of myoblast differentiation. **C**, Immunoblot of FoxO1 and Fox03 proteins in nuclear and cytoplasmic cell fractions in different stages of myoblast differentiation. **D**, FoxO1 protein levels became dramatically increased in the nuclear compartment of shTDP-43 and shFUS treated myoblasts when compared to shRNA-treated cells (n=3). **F**, Levels of nuclear FoxO3 were found slightly increased in shTDP-43-treated myoblasts, although not in shFUS-treated myoblasts (n=3). T-test, **p*<0.05, ***p*<0.01.

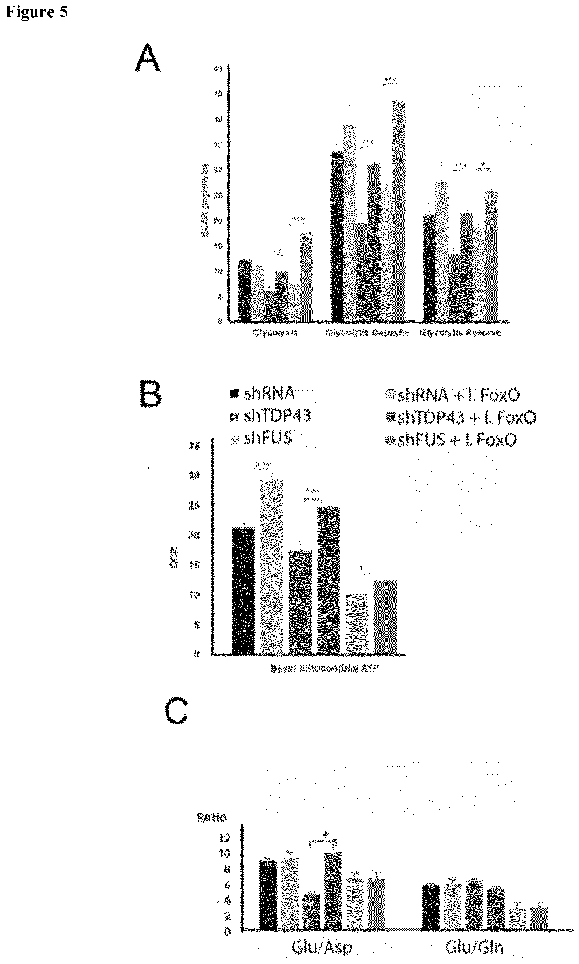

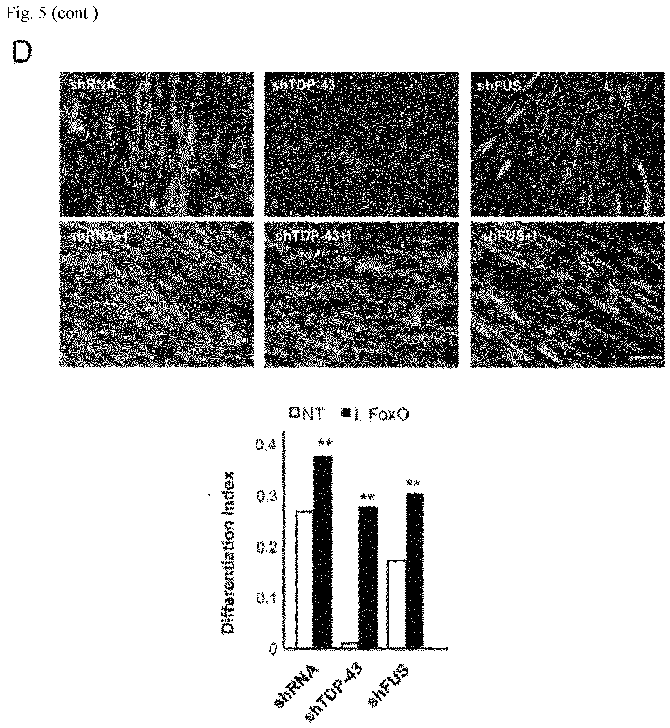

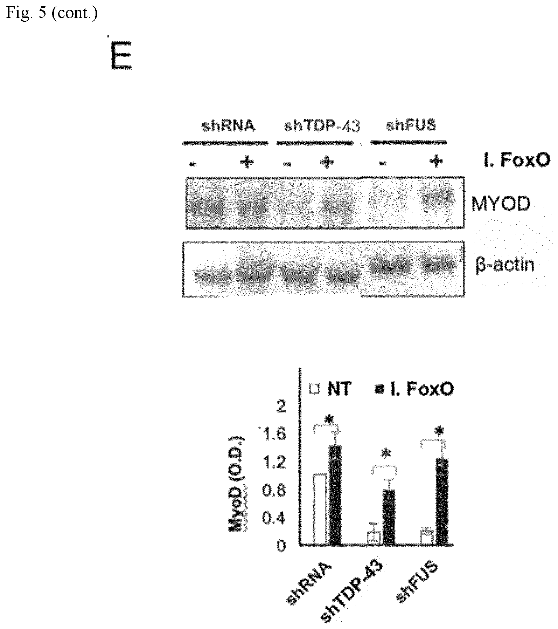

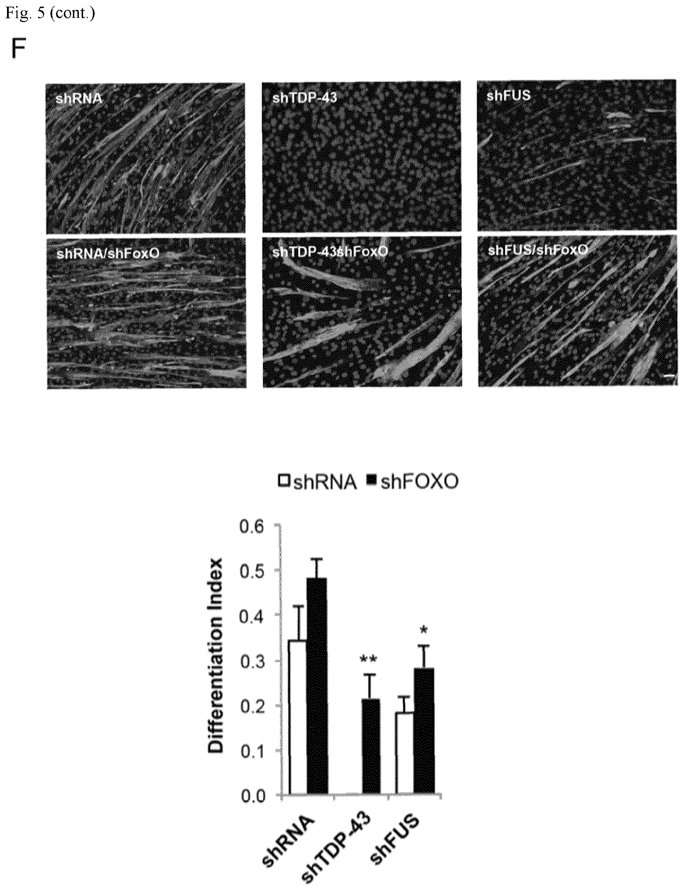

**Figure 5****. FoxO inhibition corrects metabolic derangements and myogenic defects induced by deficiency of TDP-43 or FUS**. **A-B**, Treatment with FoxO inhibitor AS1842856 at 30 nM induced a significant recovery of the repressed glycolytic traits in proliferating myoblasts caused by the silencing of either TDP-43 or FUS and enhanced mitochondrial ATP production (n=4 experiments). **C**, AS 1842856 was able to increase Glu/Asp ratio to the levels of control myoblasts in TDP-43 silenced cells without affecting Glu/Gln ratio (n=4). **D**, AS 1842856 treatment rescued the myogenic arrest of TDP-43 silenced myoblasts and improved the myogenic capacity of FUS-silenced myoblasts, as indicated by the presence of MYHC-positive (green) myotubes and the differentiation index (n=4 experiments). **E**, AS 1842856 treatment increased the expression of MYOD in shTDP-43 and shFUS-treated myoblasts (n=3 experiments). **F**, the treatment of TDP-43 and FUS-knockdown myoblasts with a shRNA sequence that targets mRNA of both FoxO1 and FoxO3 improved the myogenic capacity as well (n=3 experiments). NT, non-treated; I. FoxO, compound AS1842856 that represses the interaction of FoxO with DNA. T-test, **p*<0.05; ***p*<0.01, *** *p*<0.001.

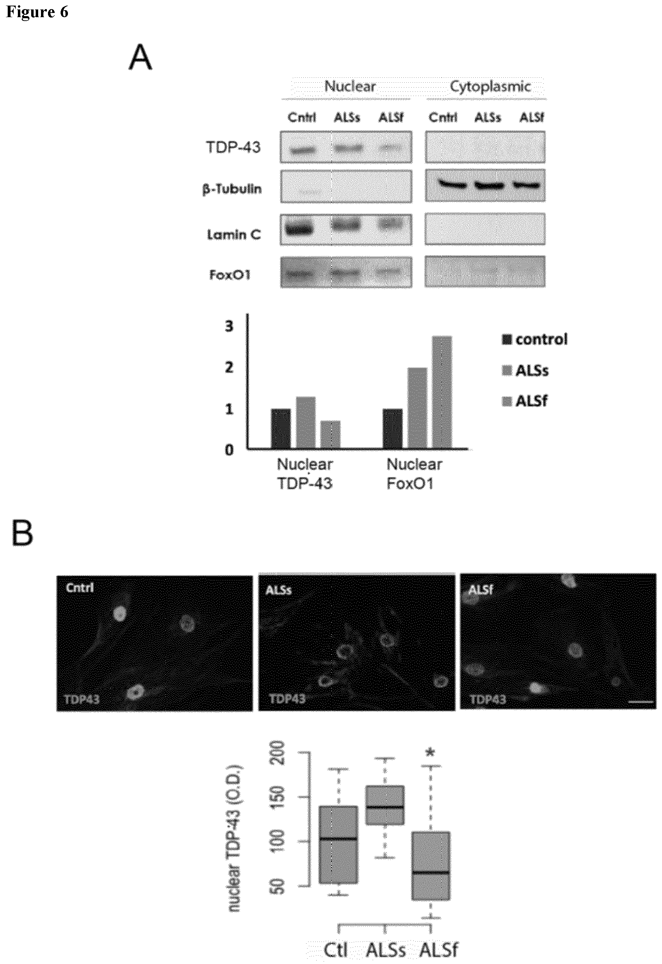

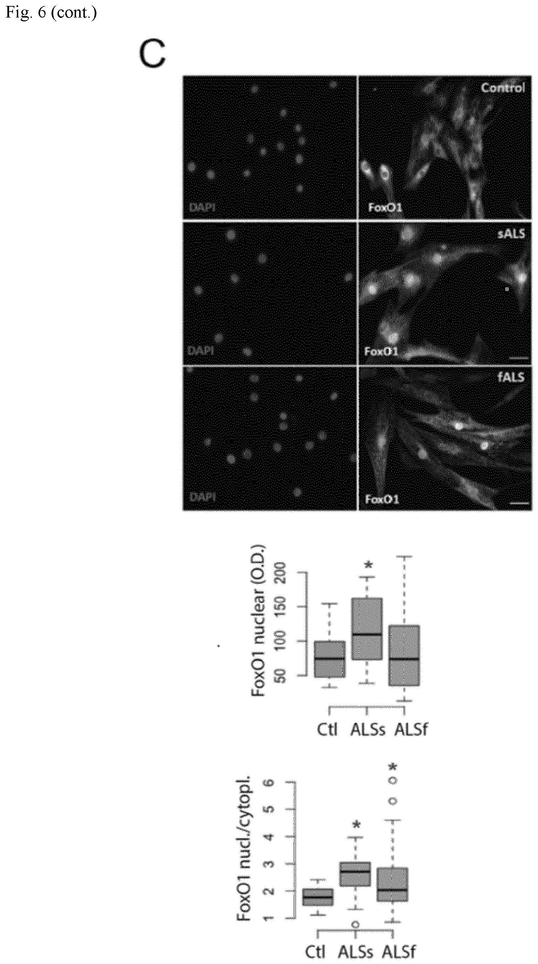

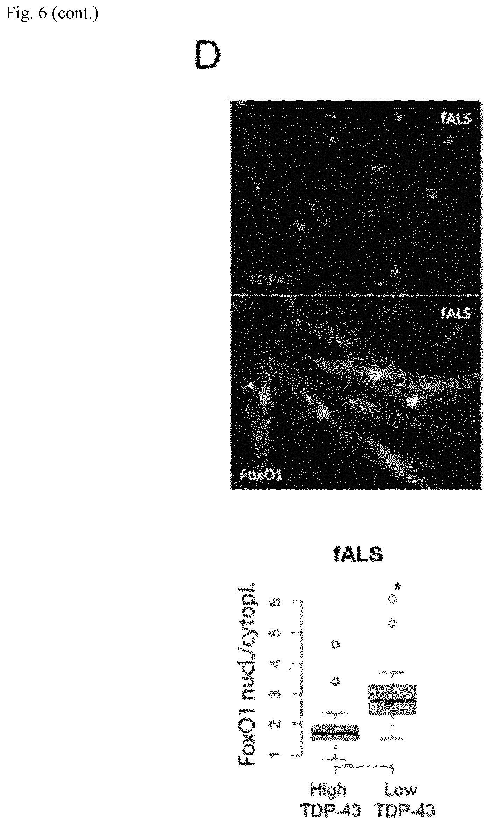

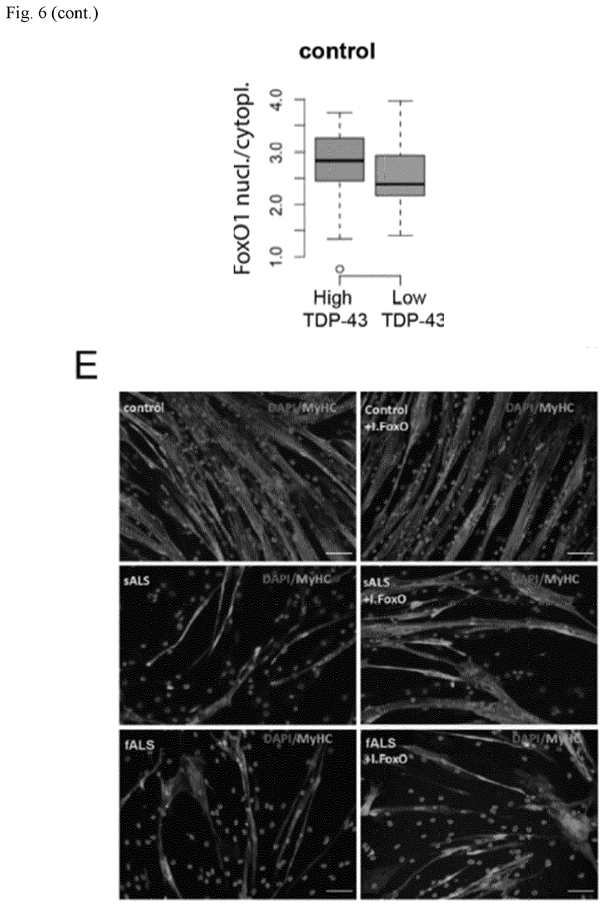

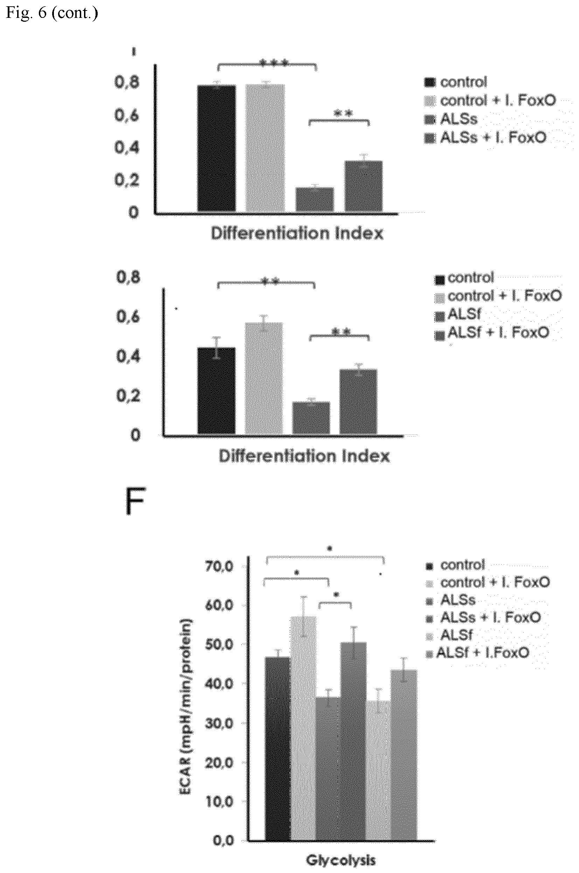

**Figure 6****. FoxO inhibition alleviates glycolytic and myogenic defects of ALS myoblasts**. **A-B**, Immunoblot analysis showed that nuclear TDP-43 were found decreased in patient 2 (ALSf, familial Amyotrophyc Lateral Sclerosis) (n=2), and immunofluorescence images showed that TDP-43 displayed a striking perinuclear localization in patient 1 (ALSs, sporadic Amyotrophyc Lateral Sclerosis). **C**, Altered subcellular distribution of FoxO1, as reflected by increased levels of FoxO1 in nuclei and enhanced nuclear-to-cytosolic ratio, in both ALS patients (n=3). **D**, Inverse correlation of nuclear TDP-43 and FoxO1 levels in a familial case of ALS (n=3 experiments). **E**, Primary myoblasts from ALS patients displayed myogenic abnormalities that were alleviated after treatment with the FoxO inhibitor AS1842856 at 30 nM (n=3 experiments). **F**, ALS myoblasts have reduced basal glycolytic flux, which was corrected treating with the FoxO inhibitor AS1842856 (n=3 experiments). T-test, **p*<0.05; ***p*<0.01, ****p*<0.001.

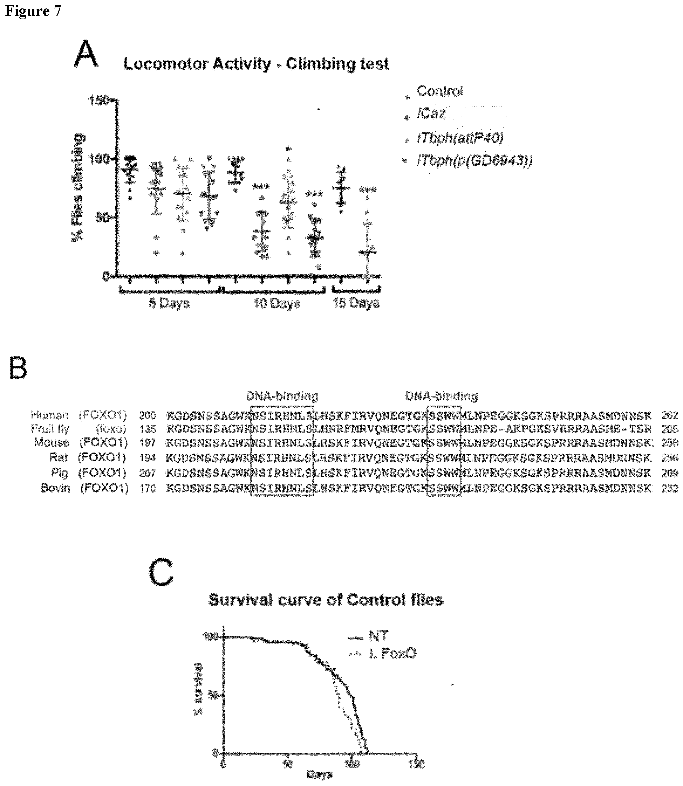

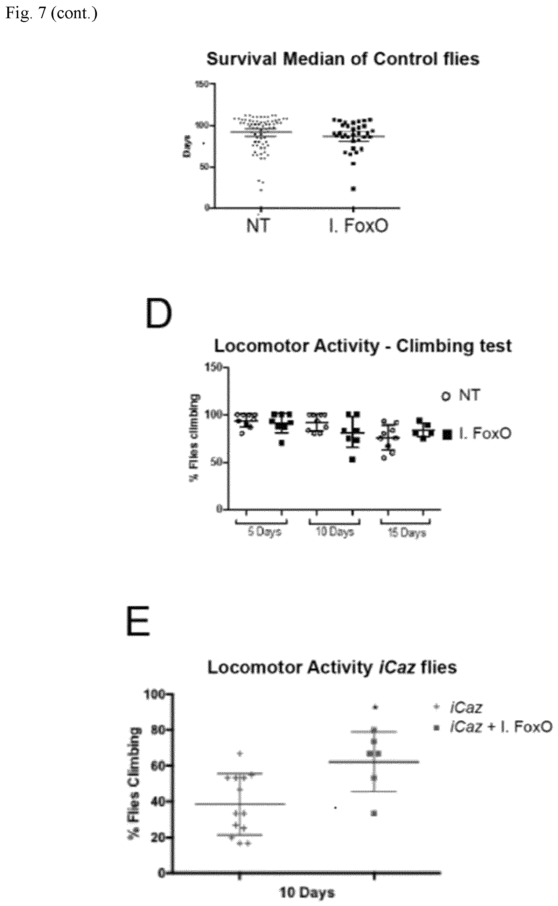

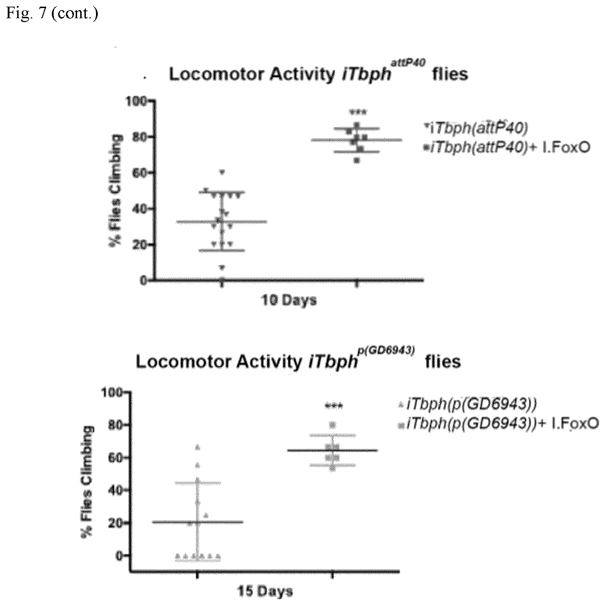

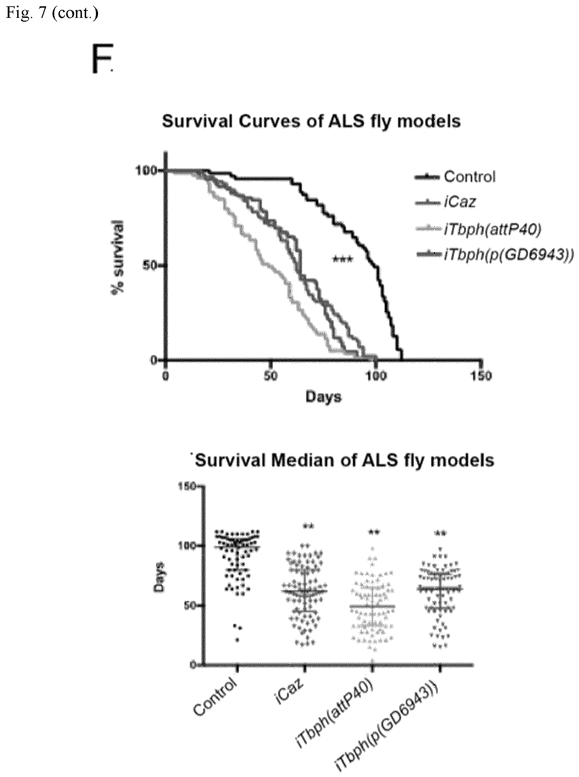

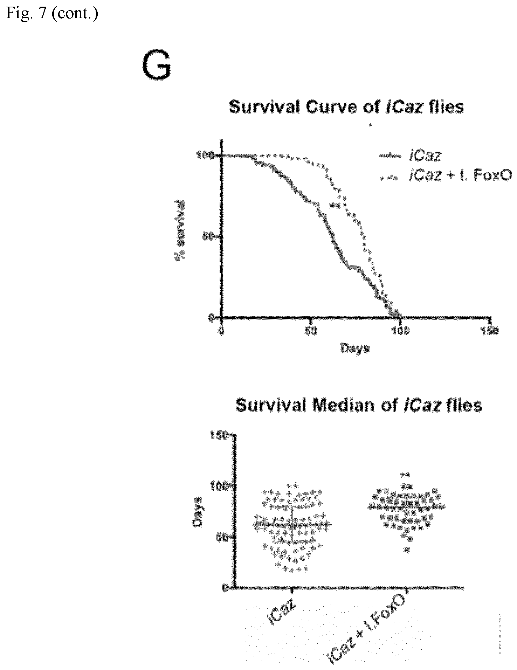

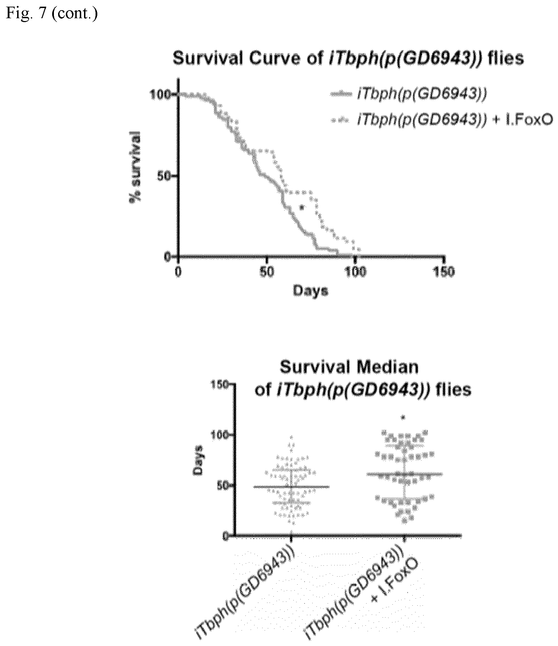

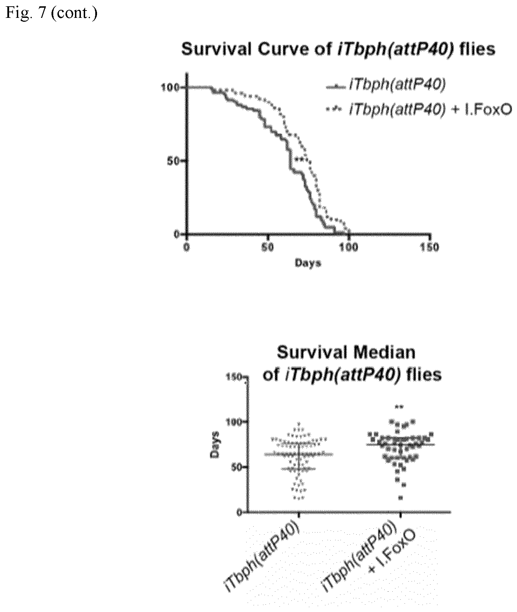

**Figure 7****. Foxo inhibition improves muscle function and lifespan in *Drosophila* flies with muscle-conditioned deficiency of TDP-43 or FUS. A**, Adult *iCaz* and *iTbph^{attp40}* flies displayed the most prominent muscle phenotypes already at 10-day old, as indicated by a significant reduction in the percentage of flies able to climb in the locomotor activity test (n=50 flies per group). Adult *iTbph^{p(GD6943}* flies showed similar degree of locomotor affection at 15-day old. **B**, FoxO DNA-binding domain, the site where AS 1842856 compound interacts with FoxO proteins, is conserved in Drosophila melanogaster. Human (SEQ ID NO: 1), Fruit fly (SEQ ID NO: 2), Mouse (SEQ ID NO: 3), Rat (SEQ ID NO: 4), Pig (SEQ ID NO: 5) and Bovin (SEQ ID NO: 6). **C**, Kaplan-Meyer curves (left panel) and median survival (right panel) of the control Drosophila strain UAS-Dicer2-+-Mef2-GAL4 with or without treatment with AS1842856 (n=100 flies per group). **D**, Climbing assay to report locomotor activity of the control Drosophila strain *UAS-Dicer2-*+*-Mef2-GAL4* with or without treatment with AS1842856 (n=50 flies per group). **E**, Treatment with AS1842856 at a concentration of 30 µM in silenced flies induced strong benefits on muscle function as observed by increased climbing activity (n=50 flies per group). **F**, Caz or Tbph-silenced flies have reduced survival times, as indicated by Kaplan-Meier curves (left panel) and median lifespan (right panel) (n=100 flies per group). **G**, Flies that were treated with AS1842856 at 30 µM had higher survival rates than flies that did not receive treatment. This effect was particularly noticeable in *iCaz* and *iTbph^{attp40}* fly lines (n=100 flies per group). **p*<0.05; ***p*<0.01, ****p*<0.001.

### Detailed description of the invention

The following examples represent the best mode of carrying out the invention, without limiting its scope.

### Example 1. Deficiency of TDP-43 or FUS halts the myogenic capacity of myoblasts.

Various lines of evidence have suggested that the myogenic program appears to be altered in patients with ALS, so we first sought to know whether these defects may be directly associated with the aberrant behavior of genes found mutated in ALS. A previous study has shown that TDP-43 is essential for normal skeletal muscle regeneration by forming cytoplasmic assemblies that regulate the expression of sarcomeric proteins. Besides TDP-43, we also focused on the study of FUS as another causative gene of ALS. We performed cell fractionation and immunofluorescence experiments throughout the different stages of myogenic differentiation in human immortalized myoblasts (cell line 8220), including: 1) proliferative myoblasts, 2) myoblast at 100% confluence (myocytes), 3) fusing myocytes, and 4) myotubes at day 3 post-fusion (**Figure 1A**). These assessments revealed changes in the subcellular localization of these proteins during early steps of myogenic differentiation despite no changes in their RNA expression levels. In particular, higher levels of TDP-43 in nuclei and cytoplasmic puncta were found in early differentiating myotubes when compared to proliferative myoblasts. In contrast, FUS was notably decreased in nuclei but increased in cytoplasm during early steps of differentiation when compared to proliferative stages (**Figure 1B****, C**).

The translocation of TDP-43 to cytoplasm is involved in myoblast differentiation and myotube generation (see reference 14). We have observed that not only TDP-43, but also FUS, suffers fluctuations of subcellular localization during myogenesis, suggesting that they both may be involved in the regulation of gene expression programs during muscle differentiation. Thus, given the potential implication of TDP-43 and FUS in myogenesis, we modelled the loss-of-function of these genes in the human immortalized myoblast cell line 8220 by treating these cells with shRNA-expressing lentiviral particles for TDP-43 or FUS, and further analyzed any change on cell morphology, growth and myogenic capacity (**Figure 1D**).

Two days after treatment with lentiviral particles, myoblasts underwent severe reductions in TDP-43 or FUS levels relative to control scramble-transduced cells (**Figure 1E**). Transmission electron microscopy (TEM) imaging revealed ultrastructure abnormalities in silenced myoblasts (**Figure 1F**). Compared to scramble-transduced myoblasts, 8820 myoblasts treated with shTDP-43 exhibited a rounded and enlarged shape with a cluster of organelles around nuclei, containing very short rough endoplasmic reticulum (RER) but lots of fragmented mitochondria. Similarly, the FUS-deficient myoblasts were found longer with abundant mitochondria although dilated RER (**Figure 1F**). Providing further evidence for increased numbers of mitochondria associated to TDP-43 and FUS deficiency, TOMM20 immunostaining showed higher mitochondrial mass in these myoblasts' lines compared to scramble-transduced control line (**Figure 1G**). Despite these changes in cellular morphology and distribution of intracellular components, 8820 myoblasts treated with either shTDP-43 or shFUS were viable, although the proliferating capacity was faintly reduced (**Figure 1H**).

We then studied the effect of gene silencing on the myogenic process. Myogenesis was first induced by an established protocol consisting on medium replacement, in which myoblasts were exposed to differentiation medium two days after infection with lentiviral particles. However, TDP-43 and FUS knockdown myoblasts were not able to survive to the differentiating challenge. Then, we switched to a more physiologically-relevant myogenic scenario that consists on allowing spontaneous differentiation and fusion when myoblasts reach 100% confluence. Lentiviral particles of scramble shRNA, shTDP-43 or shFUS were added to the medium when 8820 myoblasts were at 30-40% confluence (**Figure 1D**). Myoblasts treated with control shRNA differentiated and formed MYHC-positive myotubes 2 days after reaching 100% confluence (**Figure 1I**). However, TDP-43 and FUS knockdown affected the natural capacity of cultured myoblasts to fuse and form myotubes: shTDP-43-treated myoblasts remained undifferentiated for at least 2 days after reaching 100% confluence, and shFUS-treated myoblasts formed fewer myotubes (**Figure 1I**). At this time point, markers of myotube maturation, such as calstabin, troponin C, α-actinin and myosin, were found downregulated in shTDP-43 and shFUS treated myoblasts, corroborating defects of myogenic differentiation and fusion (**Figure 1J**). We then analyzed levels of MYOD and MYOG (Myogenin), two transcription factors that are required for myoblast commitment, differentiation and fusion, respectively. Indeed, proliferative myoblasts at 90% confluence show increased levels of MYOD with respect to differentiating myoblasts or early myotubes (**Figure 1K**); however, at that stage shTDP-43 and shFUS-treated myoblast displayed a dramatic decrease in the total levels of MYOD (**Figure 1K**) and in the number of MYOD-positive nuclei (**Figure 1L**) when compared to shRNA-treated myoblasts. Equally, Myogenin was downregulated after silencing TDP-43 and FUS (**Figure 1L**).

### Example 2. Deficiency of TDP-43 or FUS curtails anaerobic glucose metabolism and induces pro-atrophic pathways in myoblasts.

Molecular signaling factors and reprogramming of energy metabolism are considered crucial regulators of myogenesis during adult muscle regeneration. Since TDP-43 controls skeletal muscle glucose homeostasis, we wanted to investigate whether myogenic defects induced by loss of TDP-43, and in extension of FUS, may be mediated by abnormalities of glucose metabolism. Regarding the latest, various studies conducted in *Drosophila*, zebrafish and mouse myoblasts have highlighted glucose metabolism as part of the core myogenic program because of its ability to provide both the rapid source of energy and the building blocks (amino acids and nucleotides) that are required for biomass production during the formation of syncytial muscles. Thus, we investigated the extent of the metabolic reconfigurations during the myogenic process in the human myoblast cell line used in our study and, if any, whether deficiency of TDP-43 or FUS in myoblasts may impinge on these metabolic phenotypes. Later, we performed metabolic flux analysis throughout the different stages of myogenesis. Glycolysis was found to be the predominant metabolic pathway to produce basal ATP during the proliferation stages of the 8220 myoblast cell line, as indicated by higher extracellular acidification rates (ECAR) and lower oxygen consumption rates (OCR) (**Figure 2** **A,B**). However, differentiating and fusing myoblasts as well as early myotubes at day 3 underwent a switch in their metabolic potential towards a more energetic phenotype, which was due to an increase in the aerobic metabolism (**Figure 2C**). We expected that the myogenic defects induced by loss of TDP-43, and in extension of FUS, may be mediated by abnormalities of glucose metabolism.

Accordingly, we found that basal glycolysis and maximal glycolytic capacity parameters, as evidenced by ECAR, were consistently repressed after the silencing of TDP-43 or FUS in myoblasts (**Figure 2D**). Decreased levels of lactate in homogenates from shTDP-43 and shFUS-treated myoblasts when compared to those from shRNA-treated myoblasts corroborated defects in the anaerobic oxidation of glucose (**Figure 2E**). Regarding mitochondrial respiration, FUS silencing had stronger effects than TDP-43 silencing reducing basal oxygen consumption with respect to shRNA-treated myoblasts (**Figure 2F**), although spare respiratory capacity was particularly increased in shTDP-43-treated myoblasts (**Figure 2F**). The increased numbers of mitochondria, as observed by TEM and TOMM20 staining (**Figure 1F****,G**), may provide a compelling explanation for the increased aerobic potential of shTDP-43 treated myoblasts. These data indicate that the loss of TDP-43 or FUS causes general abnormalities in the energetic metabolism during the myogenic differentiation process, with specific downregulation of the glycolytic flux. A treatment with 2-deoxyglucose (2-DG), which was employed to reduce glycolysis-dependent ATP production during myoblast proliferation, reduced the activation of MYOD (**Figure 2G**) and stalled the myogenic process (**Figure 2H**) in a similar way than the silencing of TDP-43, providing further evidences of the potential link between the metabolic and myogenic phenotypes in silenced myoblasts.

A poor metabolic fitness in muscle is often associated to the upregulation of protein degradation pathways and atrophy. Expression of the key muscle specific E3 ubiquitin ligases, muscle RING finger 1 (MuRF1) and muscle atrophy F-box (MAFbx)/Atrogin-1, are markers of the activation of ubiquitin/proteasome system and atrophy in skeletal muscle. Interestingly, we observed that these proteins were significantly reduced in silenced myoblasts, particularly in shTDP-treated myoblasts (**Figure 2I**). Furthermore, the treatment with 2-DG mimicked the effects of TDP-43 and FUS silencing on the expression levels of MurF1 and Atrogin-1.

Collectively, these data support the strong energetic reliance of proliferative and differentiating 8820 myoblasts on the anaerobic glycolysis, and therefore makes this model suitable to study whether the effects of TDP-43 and FUS on myogenesis are mediated by specific impairment of this metabolic pathway.

### Example 3. Impaired NAD⁺ recycling contributes to the glycolytic defects associated to TDP-43 and FUS deficiency.

One of the key endogenous factors that keep glycolytic flux ongoing is cytosolic NAD⁺. In anaerobic muscles, malate-aspartate and glycerol-phosphate shuttles as well as glutaminolysis are main metabolic pathways that recycle cytosolic NAD⁺ to support glycolysis (**Figure 3A**). Hence, we analyzed protein levels of components of these shuttle reactions in silenced myoblasts. With respect glycerol-phosphate shuttle, levels of the cytosolic GPD1, but not mitochondrial GPD2 enzyme, were markedly reduced only in TDP-43-silenced myoblasts (**Figure 3B**). With respect malate-aspartate shuttle and glutaminolysis, TDP-43 silencing led to reduced levels of OGC1 (oxoglutarate carrier 1), GOT1 (glutamic-oxaloacetic transaminase), MDH1 (malate dehydrogenase), and ME1 (malic enzyme), but increased levels of glutaminase (**Figure 3B**). FUS silenced also induced reductions in GOT1, MDH1 and ME1 (**Figure 3B**). We have also analyzed intermediate metabolites of malate-aspartate shuttle and glutaminolysis, including glutamate (Glu), glutamine (Gln) and aspartate (Asp), by means of HPLC-MS and ¹H-NMR, and calculated the ratios Glu/Asp and Glu/Gln to estimate fluxes of these reactions. The levels of glutaminase and the Glu/Gln ratio are increased in TDP-43-silenced myoblasts when compared to control myoblasts, indicating that glutaminolysis flux may be increased under TDP-43 loss-of-function (**Figure 3C**). However, Glu/Asp ratio is dramatically decreased in TDP-43-silenced myoblasts, suggesting that Asp levels are unusually high (**Figure 3C**). The low levels of both the Asp-consuming enzymes MDH1 and GOT1, and the mitochondrial carrier OGC1 that pumps out alpha-ketoglutarate to fuel this metabolic reaction, provides a legitimate explanation for the reduced Glu/Asp ratio, and therefore suggests that malate-aspartate shuttle-dependent NAD⁺ recycling is affected in TDP-43-silenced cells. The Glu/Asp ratio is also decreased in FUS-silenced myoblasts to a similar extent than TDP-43-silenced cells (**Figure 3C**). However, the reduction in the ratio Glu/Gln and the higher levels of OGC1 suggest that a decreased glutaminolysis reaction is likely responsible for the low Glu/Asp. In this sense, an abnormal glutaminolysis may account for the defects of NAD⁺ recycling in FUS-silenced myoblasts.

### Example 4. FoxO inhibition corrects metabolic derangements and myogenic defects induced by deficiency of TDP-43 or FUS.

Because TDP-43 and FUS loss-of-function are associated with extensive changes in gene expression and splicing events, we performed microarray analysis to define the global gene expression profiles of myoblasts with TDP-43 and FUS silencing, in an attempt to find hints of gene regulation that support the observed metabolic reprogramming. The analysis of shTDP-43-transduced cells compared with control shRNA-transduced cells revealed 1,329 upregulated genes (log₂ fold change ≥1, false discovery rate [FDR]≤ 0.05, while 673 downregulated genes (log₂ fold change ≤ -1, false discovery rate [FDR] ≤ 0.05) (**Figure 4A**). The analysis of shFUS-transduced cells compared with control siRNA-transduced cells revealed 702 upregulated genes (log2 fold change ≥1, false discovery rate [FDR] ≤ 0.05); while 579 downregulated genes (log2 fold change ≤ -1, false discovery rate [FDR]≤ 0.05). Using oPOSSUM-3 (http://opossum.cisreg.ca/oPOSSUM3/), a tool for the identification of over-represented transcription factor binding sites (TFBS) in co-expressed genes generated from high-throughput methods, we generated a list of upstream transcription factors (ordered by Z-scores) that were predicted to regulate the differential gene expression between scramble shRNA and shTDP-43-treated myoblasts (**Figure 4A**). Among the top candidates predicting the differentially expressed genes (DEGs) in shTDP-43-treated myoblasts, the Forkhead Box O-3 (FoxO3) factor called our attention for mainly two reasons. First, the family of FoxO transcription factors are evolutionarily conserved mediators of insulin and growth factor signaling governing programs of gene expression to regulate apoptosis, cell-cycle, cell differentiation, metabolism and autophagy. Second, FoxO factors are involved in many types of muscle atrophy through the regulation of ligases MuRF1 and Atrogin-1, which we have found upregulated in shTDP-43 and shFUS-treated myoblasts. We performed an alternative TFBS analysis by using DAVID software (https://david.ncifcrf.gov/home.jsp), and found FoxO1 as well within the top ten list predicting TDP-43 knockdown DEGs. FoxO1 have particular implications in the regulation of multiple genes that control muscle progenitor cell maintenance and differentiation, including Myogenin. Interestingly, gene ontology (GO) analysis in shTDP-43 knockdown 8820 myoblasts revealed a significant enrichment of KEGG pathways that are upstream or downstream of FoxO transactivation, such as insulin and PI3K-AKT signaling or cell cycle (**Figure 4A**).

Taking these data together, we hypothesize that FoxO factors are underlying mediators connecting the metabolic and myogenic alterations of myoblasts with loss-of-function of either TDP-43 or FUS. If this hypothesis is correct, deficiency of TDP-43 and FUS would lead to an increased localization of FoxO factors into the nucleus and activation, while the manipulation of FoxO activities might influence the cellular phenotypes of silenced myoblasts. Regarding the subcellular localization of FoxO proteins, we observed by means of immunofluorescence and western-blotting that expression level of FoxO1 was weak and predominantly cytoplasmic during myoblast proliferation, and little bit increased in cytoplasm as well during early fusion events in control myoblasts (**Figure 4B,C****).** This is expected since pro-atrophic actions of FoxO1 must be switch off during muscle formation. However, we found that FoxO1 protein levels became dramatically increased in the nuclear compartment of shTDP-43 and shFUS treated myoblasts when compared to shRNA-treated cells (**Figure 4D**). FoxO3 was found instead highly expressed in nuclei in control myoblasts since it is needed for proliferation but decreases as differentiation starts, becoming almost exclusively cytoplasmatic in early myotubes (**Figure 4C****,E**). Levels of nuclear FoxO3 were found slightly increased in shTDP-43-treated myoblasts, although not in shFUS-treated myoblasts, when compared to control shRNA myoblasts (**Figure 4F**). Despite TDP-43 and FUS silencing induced little or no changes on the levels of nuclear FoxO3 in myoblasts, where FoxO3 is constitutively active, we cannot rule out the idea that the lack of TDP-43 or FUS may affect the shutdown of FoxO3 that is needed for myoblast differentiation.

We next explored the consequences of pharmacological or genetic inhibition of FoxO on metabolism and myogenesis in TDP-43 and FUS-silenced myoblasts. We treated all the different models of myoblasts with the compound AS 1842856 at 30 nM during proliferation and let them differentiate, adding the compound at the same concentration every 2 days for 8 days. At the selected concentration, this compound binds selectively to the dephosphorylated FoxO1, and to less extent dephosphorylated FoxO3, interfering with DNA interaction and thereby resulting in inhibition of FoxO transactivation. Interestingly, AS 1842856 treatment induced a significant recovery of the repressed glycolytic traits in proliferating myoblasts caused by the silencing of either TDP-43 or FUS (**Figure 5A**), as indicated by both glucose- and oligomycin-stimulated ECAR. This treatment also improved the mitochondrial ATP production of silenced myoblasts (**Figure 5B**), thus exerting thorough benefits in the pathways of energy production in shTDP-43 and shFUS-treated myoblasts. In the case of shTDP-43-treated myoblasts, the inhibitor AS 1842856 was able to increase Glu/Asp ratio to the levels of control myoblasts without affecting Glu/Gln ratio (**Figure 5C**). This data indicates that FoxO inhibition in shTDP-43-treated myoblast may restore the flux of malate-aspartate shuttle, which is compatible with an improved capacity of recycling NAD⁺ to fuel glycolysis.

Concomitantly with this, we observed that treatment with AS 1842856 rescued the myogenic arrest of TDP-43 silenced myoblasts and improved the myogenic capacity of FUS-silenced myoblasts, as indicated by the presence of MYHC-positive myotubes and the differentiation index (**Figure 5D**), while it had little effects on control myoblasts. In addition, treatment with the AS 1842856 inhibitor modifies the expression of some of the FoxO-regulated genes that are crucial for proper myogenic differentiation. Specifically, AS 1842856 increased the expression of MYOD in shTDP-43 and shFUS-treated 8820 myoblasts (**Figure 5E**). On top of this, the treatment of TDP-43 and FUS-knockdown myoblasts with a shRNA sequence that targets mRNA of both FoxO1 and FoxO3 was able to mitigate the abnormal nuclear expression of FoxO1 and confirmed the benefits of FoxO inhibition on the myogenic capacity of silenced myoblasts (**Figure 5F**).

### Example 5. FoxO inhibition alleviates glycolytic and myogenic defects of human ALS-derived myoblasts.

We looked at preliminary links between the findings in genetically-induced myoblasts and features of myoblasts from patients with ALS. To this aim, we cultured primary myoblasts obtained from muscle biopsies of of two patients with ALS (patient 1 is sporadic [sALS] and patient 2 is familial [fALS]) and one control. Surprisingly, when we looked at markers of ALS pathology in patients' primary myoblasts, we found clear evidences of abnormal TDP-43 localization. In particular, levels of nuclear TDP-43 were found decreased in patient 2 (**Figure 6A**), and although unchanged in patient 1, TDP-43 showed noticeably perinuclear localization (**Figure 6A****,B**). Changes in TDP-43 localization were accompanied by an altered subcellular distribution of FoxO1, as reflected by increased levels of FoxO1 and FoxO3 in nuclei (**Figure 6A****,C**) and enhanced nuclear-to-cytosolic ratio in both patients (**Figure 6C**). Remarkably, nuclear localizations of TDP-43 and FoxO1 were inversely associated in patient 2 (**Figure 6D**).

To study whether these pathological features were linked to functional outcomes of ALS muscle, we studied the myogenic capacity of ALS myoblasts and the effect of FoxO inhibition. Control primary myoblasts showed spontaneous myogenic potential, as reflected by normal fusion events and formation of mature myotubes (**Figure 6E**). In contrast, and similar to TDP-43 or FUS-silenced myoblasts, primary myoblasts from patients with ALS displayed myogenic abnormalities that were alleviated after treatment with the FoxO1 inhibitor AS 1842856 (**Figure 6E**).

As expected, alterations of FoxO1 in ALS myoblasts were accompanied by aberrant glycolytic flux, as indicated by lower ECAR values after addition of glucose to the media (**Figure 6F**). Strikingly, the treatment with the FoxO inhibitor AS 1842856 improved the basal glycolysis rate in primary ALS myoblasts concomitantly to the beneficial effects on the myogenic process (**Figure 6F**).

### Example 6. FoxO inhibition improves muscle function and lifespan in Drosophila flies with muscle-conditioned deficiency of TDP-43 or FUS.

After knowing that FoxO inhibition provides benefits to muscle cells *in vitro,* we wanted to further test the effects of targeting FoxO factors on muscle function under the context of TDP-43 or FUS loss-of-function *in vivo.* To that purpose, we generated various *Drosophila* models with muscle-conditioned silencing of either Cabeza (*Caz*) or *Tbph* genes, which are orthologs of human *FUS* or *TARDBP,* respectively. Caz-silenced (*iCaz*) flies and two different lines of Tbph-silencing (*iTbph^{attp40}* and *iTbph^{p(GD6943)}*) were viable though less than controls; however, a third line of Tbph-silencig (*iTbph^{pkk(108254)}*) did not reach adult stage despite proper development into the late pupal period. Adult *iCaz* and *iTbph^{attp40}* flies displayed the most prominent muscle phenotypes already at 10-day old, as indicated by a significant reduction in the percentage of flies able to climb in the locomotor activity test, when compared to control flies. Adult *iTbphP^{(GD6943)}* flies showed similar degree of locomotor affection at 15-day old (**Figure 7A**). *Drosophila* has only one ortholog for mammalian FoxO factors, which is called *foxo.* Although the affinity of AS 1842856 for *Drosophila foxo* has never been tested, the fact that FoxO DNA-binding sites, with which the inhibitor interacts, are well conserved among species, including Drosophila melanogaster (**Figure 7B**) provides enough rationale to test its efficacy in *Drosophila* models. We first treated control flies with AS 1842856 at a concentration of 30 µM and observed a lack of toxicity on motor function and lifespan (**Figure 7C****,D**). Interestingly, the same treatment given to silenced flies induced strong benefits on muscle function as observed by increased climbing activity, being more pronounced in Tbph-than in Caz-silenced lines (**Figure 7E**).

We performed longevity assays to further characterize these fly models. Either of the Caz or Tbph-silenced flies manifested dramatic reductions of survival times when compared to control group, as indicated by Kaplan-Meier curves and median lifespan (**Figure 7F**). Interestingly, flies that were treated with AS 1842856 had higher survival than flies that did not receive treatment. This effect was particularly noticeable in *iCaz* and *iTbph^{attp40}* fly lines (**Figure 7G**).

### REFERENCES

1. [Kierdaszuk B, et al., 2012. TDP-43 proteinopathies -from frontotemporal lobar degeneration to inclusion body myositis. Neurol Neurochir Pol. 2012 Jul-Aug;46(4):384-91. Review. Polish. PubMed PMID: 23023438].
2. [Yamazaki T, et al., 2012. FUS-SMN protein interactions link the motor neuron diseases ALS and SMA. Cell Rep. 2012 Oct 25;2(4):799-806. doi:10.1016/j.celrep.2012.08.025. Epub 2012 Sep 27. PubMed PMID: 23022481; PubMed Central PMCID: PMC3483417].
3. [Hernandez Lain A, et al., 2010. Abnormal TDP-43 and FUS proteins in muscles of sporadic IBM: similarities in a TARDBP-linked ALS patient. J Neurol Neurosurg Psychiatry. 2011 Dec;82(12):1414-6. doi: 10.1136/jnnp.2010.208868. Epub 2010 Jun 18. PubMed PMID: 20562395].
4. [Greenberg SA. Inflammatory myopathies: disease mechanisms. Curr Opin Neurol.2009 Oct;22(5):516-23. doi: 10.1097/WCO.0b013e3283311ddf. Review. PubMed PMID: 19680126].
5. [Banno H, ET AL., 2009. Molecular-targeted therapy for motor neuron disease. Brain Nerve. 2009 Aug;61(8):891-900. Review. Japanese. PubMed PMID: 19697878].
6. [Tan CF, ET AL., 2009. Selective occurrence of TDP-43-immunoreactive inclusions in the lower motor neurons in Machado-Joseph disease. Acta Neuropathol. 2009 Oct;118(4):553-60. doi:10.1007/s00401-009-0552-x. Epub 2009 Jun 13. PubMed PMID: 19526244].
7. [Küsters B, ET AL., 2008. TDP-43 accumulation is common in myopathies with rimmed vacuoles. Acta Neuropathol. 2009 Feb; 117(2):209-11. doi: 10.1007/s00401-008-0471-2. Epub 2008 Dec 9. PubMed PMID: 19066918].
8. [Llamusi B, ET AL., 2013. Muscleblind, BSF and TBPH are mislocalized in the muscle sarcomere of a Drosophila myotonic dystrophy model. Dis Model Mech. 2013 Jan;6(1):184-96. doi: 10.1242/dmm.009563. Epub 2012 Nov 1. PubMed PMID: 23118342; PubMed Central PMCID: PMC3529350].
9. [Papadimas GK, ET AL., 2016. GNE-Myopathy in a Greek Romani Family with Unusual Calf Phenotype and Protein Aggregation Pathology. J Neuromuscul Dis. 2016 May 27;3(2):283-288. PubMed PMID: 27854221].
10. [Homma S, et al., 2015. Expression of FSHD-related DUX4-FLalters proteostasis and induces TDP-43 aggregation. Ann Clin Transl Neurol. 2015 Feb;2(2):151-66. doi: 10.1002/acn3.158. Epub 2015 Jan 15. PubMed PMID: 25750920; PubMed Central PMCID: PMC4338956].
11. [Palmio J, et al., 2020. Mutations in the J domain of DNAJB6 cause dominant distal myopathy. Neuromuscul Disord. 2020 Jan;30(1):38-46. doi:10.1016/j.nmd.2019.11.005. Epub 2019 Nov 19. PubMed PMID: 31955980].
12. [Mori F, et al., 2019. Phosphorylated TDP-43 aggregates in skeletal and cardiac muscle are a marker of myogenic degeneration in amyotrophic lateralsclerosis and various conditions. Acta Neuropathol Commun. 2019 Oct 28;7(1):165. doi: 10.1186/s40478-019-0824-1. PubMed PMID: 31661037; PubMed Central PMCID:PMC6816170].
13. [Crociara P, et al., 2019. Motor neuron degeneration, severe myopathy and TDP-43 increase in a transgenic pig model of SOD1-linked familiar ALS. Neurobiol Dis.2019 Apr;124:263-275. doi: 10.1016/j.nbd.2018.11.021. Epub 2018 Nov 22. PubMedPMID: 30471417].
14. [Vogler TO, et al., 2018. TDP-43 and RNA form amyloid-like myo-granules in regenerating muscle. Nature. 2018 Nov;563(7732):508-513. doi: 10.1038/s41586-018-0665-2. Epub 2018 Oct 31. PMID: 30464263 PMCID: PMC6324568 DOI: 10.1038/s41586-018-0665-2].

## Claims

1. FoxO inhibitor for use in the treatment of neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

2. FOXO inhibitor for use, according to claim 1, wherein the FoxO inhibitor is a quinolone derivative selected from the group comprising:

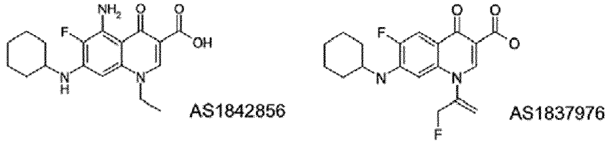

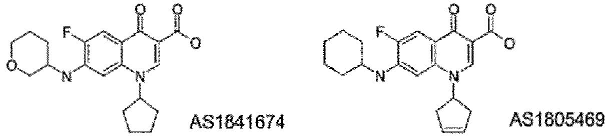

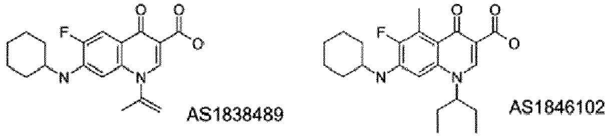

3. FoxO inhibitor AS 1842856 for use, according to any of the previous claims, in the treatment of neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

4. FoxO inhibitor AS 1842856 for use, according to any of the previous claims, in the treatment of Amyotrophic Lateral Sclerosis.

5. Pharmaceutical composition comprising FoxO inhibitors and, optionally, pharmaceutically acceptable excipients or carriers, for use in the treatment of neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

6. Pharmaceutical composition for use, according to claim 5, wherein the FoxO inhibitor is a quinolone derivative selected from the group comprising:

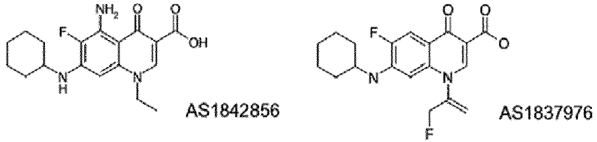

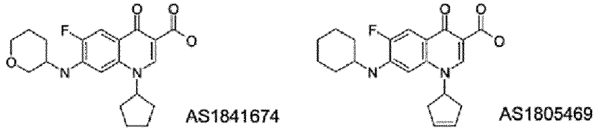

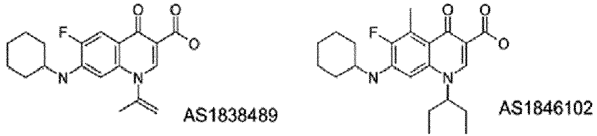

7. Pharmaceutical composition for use, according to any of the claims 5 or 6, comprising the FoxO inhibitor AS1842856 and, optionally, pharmaceutically acceptable excipients or carriers.

8. Pharmaceutical composition comprising the FoxO inhibitor AS 1842856 for use, according to any of the claims 5 to 7, in the treatment of Amyotrophic Lateral Sclerosis.

9. *In vitro* method for the identification and production of candidate compounds for the treatment of neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, comprising: a) determining whether FoxO has been inhibited after administering the candidate molecule, b) where, if after administering the candidate molecule, FoxO has been inhibited, this is indicative that the candidate molecule is effective in the treatment of neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

10. *In vitro* method, according to claim 9, for the identification and production of candidate compounds for the treatment of Amyotrophic Lateral Sclerosis, comprising: a) determining whether FoxO has been inhibited after administering the candidate molecule, b) where, if after administering the candidate molecule, FoxO has been inhibited, this is indicative that the candidate molecule is effective in the treatment of Amyotrophic Lateral Sclerosis.

11. *In vitro* method for monitoring the response to a treatment against neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, comprising: a) determining whether FoxO has been inhibited after administering the treatment, b) where, if after administering the treatment, FoxO has been inhibited, this is indicative that the therapy is effective in the treatment of neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

12. *In vitro* method, according to claim 11, for monitoring the response to a treatment against Amyotrophic Lateral Sclerosis, comprising: a) determining whether FoxO has been inhibited after administering the treatment, b) where, if after administering the treatment, FoxO has been inhibited, this is indicative that the treatment is effective in the treatment of Amyotrophic Lateral Sclerosis.

13. *In vitro* method for the diagnosis of neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, which comprises measuring the level of expression of FoxO in a biological sample obtained from a patient, wherein a variation or deviation of the level of expression of FoxO measured in the patient with respect to the level measured in healthy control patients, is indicative that the patient is suffering from a neuromuscular and/or neurodegenerative disorder selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

14. *In vitro* method for the diagnosis of neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, which comprises measuring the level of expression of NAD⁺ or NAD⁺/NADH index in a biological sample obtained from a patient, wherein a reduction of the level of expression of NAD⁺ or NAD⁺/NADH index measured in the patient with respect to the level measured in healthy control patients, is indicative that the patient is suffering from a neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

15. *In vitro* method for predicting or monitoring the response to a treatment of neuromuscular and/or neurodegenerative disorders selected from the group comprising: Amyotrophic lateral sclerosis, Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy, which comprises measuring the level of expression of NAD⁺ or NAD⁺/NADH index in a biological sample obtained from a patient after the treatment, wherein an increase of the level of expression of NAD⁺ or NAD⁺/NADH index measured in the patient with respect to the level measured in control patients, it is indicative that the treatment is effective.
